# EUROPEAN PATENT APPLICATION

(11) **EP 4 458 295 A1**
(43) Date of publication of application: **06.11.2024**
(21) Application number: 22914899.4
(22) Date of filing: 28.12.2022
(51) Int. Cl.: A61B 18/00, A61B 18/12, A61B 18/14

(54) **ABLATION APPARATUS**

(30) Priority: 31.12.2021 CN 202111674234; 31.12.2021 CN 202111677701
(71) Applicant: Shenzhen Lifetech Respiration Scientific Co., Ltd., Shenzhen, Guangdong 518057 (CN)
(72) Inventor: HU, Longhu, Shenzhen, Guangdong 518063 (CN); SHI, Yue, Shenzhen, Guangdong 518063 (CN); LI, Anning, Shenzhen, Guangdong 518063 (CN)
(74) Representative: Prinz & Partner mbB
(86) International application number: PCT/CN2022/142753
(87) International publication number: WO 2023/125647

(57) **Abstract**

An ablation apparatus (20) is provided, including: a catheter (21) and an ablation portion (22) connected to a distal end of the catheter (21), where the ablation portion (22) has an expansible property and includes a plurality of ablation members (22a); each ablation member (22a) includes a supporting unit (24), a wall attachment unit (23), and an energy release unit provided on the wall attachment unit (23); a plurality of supporting units (24) are deployed radially outwardly and extend toward the distal end of the ablation portion (22) to be connected to a plurality of wall attachment units (23); when the ablation portion (22) is in a naturally expanded state, at least one wall attachment unit (23) in the ablation portion (22) is independently deformed relative to the adjacent wall attachment units (23). The ablation apparatus (20) can adapt to the morphology of a target tissue and attach well to the target tissue.

## Description

### Cross-Reference to Related Application

This application claims priority to Chinese Patent Application No. 2021116742340, entitled "Cooling Balloon and Ablation Apparatus" filed to China National Intellectual Property Administration on December 31, 2021, and Chinese Patent Application No. 2021116777015, entitled "Ablation Apparatus" filed to China National Intellectual Property Administration on December 31, 2021, the entire contents of which are incorporated herein by reference.

### Technical Field

The present disclosure belongs to the technical field of medical devices, and particularly relates to an ablation apparatus.

### Background Art

Chronic obstructive pulmonary disease (COPD) is currently the most common type of chronic airway disease, which may seriously affect the quality of life of patients and is an important cause of death. Its main features are persistent airflow limitation and corresponding respiratory symptoms, and the main pathological manifestations are airway and/or alveolar abnormalities. As the disease progresses, dyspnea is noted during non-strenuous activities such as walking. Over time, symptoms of COPD may develop with decreasing activity until they occur at all times, severely limiting a person's ability to perform normal activities. Pulmonary disease often has the features of obstruction of the airway lumen, thickening of the airway wall, changes of structures within or around the airway wall, or a combination thereof. Airway obstruction may significantly reduce the amount of gas exchange in the lungs resulting in dyspnea. Obstruction of the airway lumen may be caused by excessive intraluminal mucus or edematous fluid or both. Thickening of the airway wall may be caused by excessive shrinkage of airway smooth muscle, airway smooth muscle hypertrophy, mucus gland hyperplasia, inflammation, edema, or a combination thereof. Changes of structures around the airway, such as destruction of the lung tissue itself, may result in loss of radial shrinkage of the airway wall and subsequent airway stenosis. Asthma and COPD are serious diseases with an increasing number of patients.

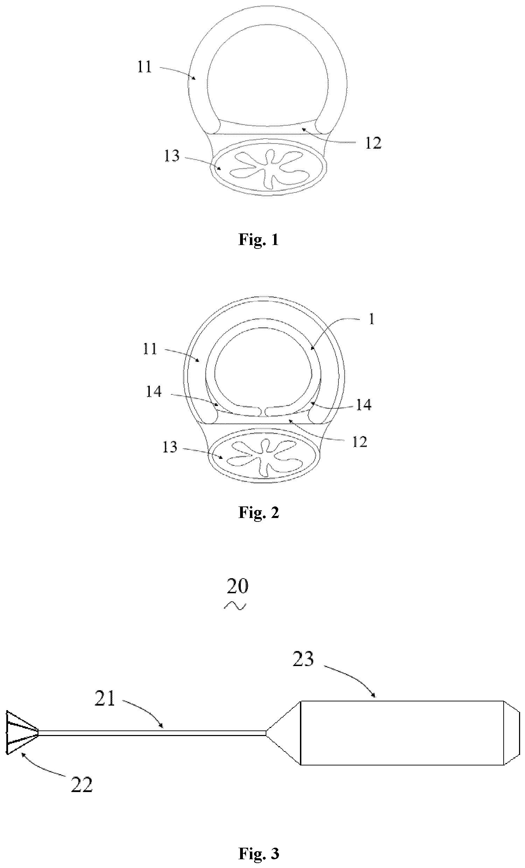

As a new trend to treat COPD in recent years, targeted lung denervation (TLD) mainly releases ablation energy through an ablation apparatus to ablate parasympathetic nerves in the bronchial adventitia, thereby blocking the transmission of nerve signals, relaxing the airway smooth muscle, and reducing mucus secretion, thus improving the symptoms of airway obstruction and dyspnea. Referring to Fig. 1, it shows a typical anatomical structure of an airway (also known as a trachea). The airway mainly includes cartilages, smooth muscle fibers, and connective tissues. A cartilage 11 of the airway is "C" shaped with a notch facing an esophagus 13. Generally, there are 14-16 cartilages, 11 in the airway, which are arranged along a longitudinal direction of the airway at intervals and connected to each other by annular ligaments. A dorsal muscular wall portion 12 formed by the smooth muscle fiber and the connective tissue closes the notch of the cartilage 11. The dorsal muscular wall portion 12 is relatively flat so that the cross-section of an inner wall of the airway is substantially "D" shaped. A C-shaped region accounts for about two thirds of the circumference of the cross-section of the airway, and the relatively flat dorsal muscular wall portion 12 accounts for about one third of the circumference of the cross-section of the airway. Fig. 2 shows a schematic transverse cross-sectional diagram of an existing ablation apparatus deployed in an airway. The ablation apparatus includes a catheter and an electrode (not shown in the figure) provided at a distal end of the catheter. The distal end of the catheter of the ablation apparatus forms an annular structure 1 with an opening. The annular structure 1 may be deformed to a certain extent under radial pressure of the inner wall of the airway due to the opening. However, as the annular structure 1 is an integral structure, deformation in a certain region of the annular structure 1 due to the radial pressure inevitably drives the adjacent region to deform. Consequently, the annular structure 1 cannot attach properly to a target tissue with a non-circular profile, and thus the ablation energy of the electrode located on the annular structure 1 cannot be effectively transmitted to the target tissue.

### Summary of the Disclosure

It is an object of the present disclosure to provide an ablation apparatus that can adapt to the morphology of a target tissue and attach well to the target tissue.

The object is achieved in the following ways.

A first aspect of the present disclosure provides an ablation apparatus, including: a catheter and an ablation portion connected to a distal end of the catheter, where the ablation portion has an expansible property and includes a plurality of ablation members; each ablation member includes a supporting unit, a wall attachment unit, and an energy release unit provided on the wall attachment unit; a plurality of supporting units are deployed radially outwardly and extend toward the distal end of the ablation portion to be connected to a plurality of wall attachment units; when the ablation portion is in a naturally expanded state, at least one wall attachment unit in the ablation portion is independently deformed relative to the adjacent wall attachment units.

In one embodiment, the plurality of supporting units is enclosed to form a cavity having an opening at the distal end, and the plurality of wall attachment units are provided around the opening at intervals.

In one embodiment, the wall attachment unit includes a first connecting end and a second connecting end connected to the supporting unit, and a deformable section extending between the first connecting end and the second connecting end; the deformable section has an elastic deformation property, and when the ablation portion is in the naturally expanded state, the deformable section is bent and protrudes toward an outer side of the ablation portion relative to the first connecting end and the second connecting end.

In one embodiment, the deformable section is elastically deformed to increase the distance between the first connecting end and the second connecting end when the deformable section is subjected to a force toward an inside of the ablation portion.

In one embodiment, the deformable section includes a first wall attachment section and a second wall attachment section; one end of the first wall attachment section is connected to the first connecting end, the other end of the first wall attachment section is connected to one end of the second wall attachment section, and the other end of the second wall attachment section is connected to the second connecting end; the first connecting end and the second connecting end are configured to be close to each other under the action of an external force and away from each other when the external force is removed.

In one embodiment, the first wall attachment section and the second wall attachment section each includes an arcuate structure protruding toward the outer side of the ablation portion, or the first wall attachment section and the second wall attachment section each includes a straight-line structure inclined toward the outer side of the ablation portion; or, one of the first wall attachment section and the second wall attachment section includes the arcuate structure protruding toward the outer side of the ablation portion, and the other includes the straight-line structure inclined toward the outer side of the ablation portion.

In one embodiment, the deformable section further includes a connecting section; the connecting section includes a distal vertex, and a first connecting arm and a second connecting arm jointly connecting the distal vertex; a gap exists between a proximal end of the first connecting arm and a proximal end of the second connecting arm; one end of the first wall attachment section is connected to the first connecting end, and the other end of the first wall attachment section is connected to the first connecting arm; one end of the second wall attachment section is connected to the second connecting arm, and the other end of the second wall attachment section is connected to the second connecting end.

In one embodiment, the connecting section is protruded toward the distal end of the ablation apparatus.

In one embodiment, the supporting unit includes two first supporting rods provided at intervals, and the first connecting end and the second connecting end are fixedly connected to the two first supporting rods, respectively.

In one embodiment, the ablation portion further includes a supporting member; the supporting member includes a plurality of second supporting rods arranged at spaced intervals; the proximal end of each second supporting rod is fixedly connected to the catheter, and the distal end of each second supporting unit is fixedly connected to two adjacent first supporting units.

In one embodiment, the supporting unit includes a straight section and is connected to the wall attachment unit through the straight section; the included angle between the straight section and the axis of the ablation portion is less than the included angle between a remaining region of the supporting unit except the straight section and the axis of the ablation portion in a naturally expanded state.

In one embodiment, the ablation apparatus has a first region and a second region; the wall attachment unit in the first region is independently deformed relative to the adjacent wall attachment units, and the wall attachment units in the first region and the wall attachment units in the second region are located on the same plane, or the wall attachment units in the first region and the wall attachment units in the second region are located in different planes.

In one embodiment, the wall attachment units in the first region are located between the wall attachment units in the second region and the catheter, and when both the first region and the second region are subjected to a force toward an inner side of the ablation portion, at least one wall attachment unit in the second region abuts against the inside of the wall attachment unit in the first region.

In one embodiment, the energy release unit located in the first region includes an electrode pair and/or strip electrodes; the electrode pair includes a first electrode and a second electrode of opposite polarities, and the first electrode and the second electrode are provided at intervals on one or more wall attachment units; the strip electrodes extend along a longitudinal direction of the wall attachment unit.

In one embodiment, the wall attachment unit includes a sheet-like mesh structure; the mesh structure includes conductive wires, and the conductive wire is exposed to form a mesh electrode.

In one embodiment, the catheter and the ablation portion have delivery channels that communicate with each other; and/or the distal end of the catheter is provided with an extension tube, the extension tube passes through the ablation portion, and the catheter and the extension tube are provided with delivery channels communicated with each other; first cooling holes are arranged on a surface of the ablation portion and/or the extension tube, and the first cooling hole communicates the delivery channel with the outside; the delivery channel is configured to deliver a cooling medium, and the first cooling hole is configured to release the cooling medium to the outside.

In one embodiment, the ablation apparatus further includes a cooling balloon; the cooling balloon includes a balloon main body having a balloon chamber, a delivery channel communicates with the balloon chamber; the balloon main body is provided in a chamber of the ablation portion, and when the balloon main body is in an expanded state, the outer surface of the balloon main body is attached to the wall attachment unit; the delivery channel is configured to deliver a cooling medium into the balloon chamber; one or more second cooling holes are arranged on a surface of the cooling balloon and are configured to communicate the balloon chamber with the outside and discharge the cooling medium in the balloon chamber to the outside.

In one embodiment, the cooling balloon further includes a recovery channel that communicates with the balloon chamber, and the recovery channel is configured to recover the cooling medium from the balloon chamber.

The present disclosure also provides a cooling balloon, including: an expandable balloon main body, a delivery channel, and a recovery channel, where the balloon main body includes a first balloon chamber and a second balloon chamber arranged in a radial direction, and the delivery channel, the recovery channel, the first balloon chamber, and the second balloon chamber form a cooling circuit for a cooling medium to flow; the first balloon chamber has a first outer wall, the second balloon chamber has a second outer wall, and the first outer wall and the second outer wall are arranged in a circumferential direction of the balloon main body; any cross-sectional area of the first balloon chamber in an axial region where the first outer wall is located is a first cross-sectional area, any cross-sectional area of the second balloon chamber in an axial region where the second outer wall is located is a second cross-sectional area, and the first cross-sectional area is greater than the second cross-sectional area.

In one embodiment, the cooling balloon further includes an outer tube, a separating piece, and an output hole; the distal end of the outer tube is connected to the proximal end of the balloon main body; the outer tube is a multi-chamber tube, and the interior of the outer tube has the delivery channel and the recovery channel independent of each other; one of the first balloon chamber and the second balloon chamber communicates with the distal end of the delivery channel, and the other is communicated with the distal end of the recovery channel; the proximal end of the delivery channel is configured to connect a cooling apparatus, and the proximal end of the recovery channel is configured to connect a recovery apparatus; the separating piece is fixedly connected to the interior of the balloon main body and is provided between the first balloon chamber and the second balloon chamber, configured to separate the first balloon chamber and the second balloon chamber; at least one output hole is arranged between the first balloon chamber and the second balloon chamber, and the output hole communicates the first balloon chamber and the second balloon chamber and is configured to flow the cooling medium between the first balloon chamber and the second balloon chamber.

In one embodiment, the separating piece includes a laminar structure; the laminar structure extends from the proximal end to the distal end of the balloon main body to separate the first balloon chamber and the second balloon chamber, and the cross-sectional area occupied by the first balloon chamber is greater than the cross-sectional area occupied by the second balloon chamber in any cross-section of the balloon main body in an axial region where the separating piece is located.

In one embodiment, the separating piece further includes an inner balloon, and a cooling cavity is formed between the outer surface of the inner balloon and the inner surface of the balloon main body; the laminar structure is located in the cooling cavity and separates the first balloon chamber and the second balloon chamber from the cooling cavity.

In one embodiment, the cooling balloon further includes an inner rod; an accommodating passage is also provided in the outer tube; the inner rod extends through the accommodating passage, and the distal end of the inner rod extends into the balloon chamber and is fixedly connected to the distal end of the balloon main body.

In one embodiment, the output hole is located on the separating piece and/or on an inner rod, and the distance from the output hole to the distal end of the balloon main body is smaller than the distance from the output hole to the proximal end of the balloon main body.

In one embodiment, the delivery channel includes a first delivery channel and a second delivery channel, and the recovery channel includes a first recovery channel and a second recovery channel; the first delivery channel, the first recovery channel, and the first balloon chamber form a first cooling circuit, and the second delivery channel, the second recovery channel, and the second balloon chamber form a second cooling circuit; when the first balloon chamber and the second balloon chamber are in an expanded state, the flow rate of the cooling medium in the axial region where the second outer wall of the second balloon chamber is located is greater than the flow rate of the cooling medium in the axial region where the first outer wall of the first balloon chamber is located.

In one embodiment, the surface roughness of an inner surface of the second outer wall is less than the surface roughness of the inner surface of the first outer wall.

In one embodiment, the outer surface hardness of the second outer wall is less than the outer surface hardness of the first outer wall.

In one embodiment, the balloon main body is provided with a cooling pipe, the concentration of the cooling pipe on the first outer wall is less than the concentration of the cooling pipe on the second outer wall.

The present disclosure also provides an ablation apparatus, including the cooling balloon according to any of the above and an ablation portion; the ablation portion is configured to ablate a target tissue; the cooling balloon is inserted into the ablation portion and is configured to cool the target tissue.

In the ablation apparatus provided by the present disclosure, at least one wall attachment unit in the ablation portion is a deformable unit, and when the ablation portion is deployed in an airway, the deformable unit may be independently deformed relative to the adjacent wall attachment units when the deformable unit is subject to a squeezing force by an inner wall of the airway, so as to avoid interference with the adjacent wall attachment units when the deformable unit is deformed, thereby enabling the regions of the ablation portion to better adapt to the morphology of the inner wall of the airway to deform, and improving the attachment of the ablation apparatus to the target tissue.

### Brief Description of the Drawings

Various other advantages and benefits will become clear to a person skilled in the art by reading the detailed description of the preferred implementations below. The accompanying drawings are only for purposes of illustrating the preferred implementations and are not to be construed as limiting the present disclosure. Moreover, the same reference numeral represents the same part throughout the drawings, in which:
Fig. 1 is a typical anatomical structure of an airway;
Fig. 2 is a schematic diagram of an ablation apparatus deployed in an airway in the related art;
Fig. 3 is a schematic structural diagram of an ablation apparatus according to embodiment 1 of the present disclosure;
Fig. 4 is a schematic structural diagram of an ablation portion of Fig. 3 in a naturally expanded state;
Fig. 5 is a side view of the ablation portion of Fig. 4;
Fig. 6 is a schematic diagram of the deformation of the ablation portion of Fig. 4 under force;
Fig. 7 is a schematic diagram of the deformation of a wall attachment unit of Fig. 4 under force;
Fig. 8 is a schematic diagram of an electrode arrangement of the ablation portion of Fig. 4;
Fig. 9 is a schematic diagram of another electrode arrangement of the ablation portion of Fig. 4;
Fig. 10 is a partial schematic structural diagram of an ablation apparatus according to embodiment 2 of the present disclosure;
Fig. 11 is a schematic diagram of a deformation of an ablation portion of Fig. 10 under force;
Fig. 12 is a partial schematic structural diagram of an ablation apparatus according to embodiment 3 of the present disclosure;
Fig. 13 is a partial schematic structural diagram of an ablation apparatus according to embodiment 4 of the present disclosure;
Fig. 14 is a schematic diagram of a cross-section of an ablation member of Fig. 13;
Fig. 15 is a schematic diagram of a cross-section of a supporting member of Fig. 13;
Fig. 16 is a partial schematic structural diagram of an ablation apparatus according to another embodiment of the present disclosure;
Fig. 17 is a partial schematic structural diagram of an ablation apparatus according to embodiment 5 of the present disclosure;
Fig. 18 is a schematic structural diagram of a cooling balloon of Fig. 17;
Fig. 19 is a partial schematic structural diagram of an ablation apparatus according to another embodiment of the present disclosure;
Fig. 20 is a partial schematic structural diagram of a cooling balloon according to embodiment 6 of the present disclosure;
Fig. 21 is a partial schematic structural diagram of a cooling balloon according to embodiment 7 of the present disclosure;
Fig. 22 is a partial schematic structural diagram of a cooling balloon according to another embodiment of the present disclosure;
Fig. 23 is a partial schematic structural diagram of a cooling balloon according to embodiment 8 of the present disclosure;
Fig. 24 is a schematic diagram of the cross-section of a middle region of a cooling balloon according to another embodiment of the present disclosure;
Fig. 25 is a partial schematic structural diagram of a cooling balloon according to embodiment 9 of the present disclosure; and
Fig. 26 is a schematic diagram of plane deployment of a region where an encircling section of the cooling balloon of Fig. 25 is located.

### Detailed Description of the Disclosure

Exemplary implementations of the present disclosure will be described in more detail below with reference to the accompanying drawings. Although exemplary implementations of the present disclosure are shown in the accompanying drawings, it should be understood that the present disclosure may be implemented in various forms and should not be limited by the implementations described herein. Rather, these implementations are provided so that the present disclosure will be thoroughly understood, and the scope of the present disclosure will be fully conveyed to a person skilled in the art.

It should be understood that the terms used herein are for the purpose of describing particular example implementations only and are not intended to be limiting. As used herein, the singular forms "a", "an", and "the" are intended to include the plural forms as well, unless the context clearly indicates otherwise. The terms "comprise", "include", "contain", and "have" are inclusive, and therefore indicate the presence of the stated features, steps, operations, elements, and/or components, but do not exclude the presence or addition of one or more other features, steps, operations, elements, components, and/or their combinations. The method steps, processes, and operations described herein are not to be interpreted as requiring them to be performed in the specific order described or illustrated, unless a specific order of performance is explicitly stated. It should also be understood that additional or alternative steps may be used.

Although the terms first, second, third, etc. may be used herein to describe a plurality of elements, components, regions, layers, and/or sections, these elements, components, regions, layers, and/or sections should not be limited by these terms. These terms may only be used to distinguish one element, component, region, layer, or section from another region, layer, or section. The use of terms such as "first", "second", and other numerical terms herein does not imply a sequence or order unless the context clearly dictates otherwise. Thus, a first element, component, region, layer, or section discussed below may be referred to as a second element, component, region, layer, or section without departing from the teachings of the example implementations.

For ease of description, spatially relative terms, such as "interior", "outside", "inner side", "outer side", "below", "under", "above", "top", and the like, may be used herein to describe one element or feature's relationship to another element or feature as illustrated in the drawings. Such spatially relative terms are intended to include different orientations of an apparatus in use or operation in addition to the orientation depicted in the drawings. For example, if the apparatus in the drawing is flipped, elements described as "under other elements or features" or "beneath other elements or features" would then be directed as "over other elements or features" or "above other elements or features". Therefore, the exemplary term "below" may include orientations of above and below. The apparatus may be otherwise oriented (rotated 90 degrees or in other directions) and interpreted accordingly by the spatially relative descriptors used herein.

To more clearly describe a structure of an ablation apparatus, the terms "proximal end" and "distal end" are defined herein as conventional terms used in the interventional medical art. Specifically, "distal end" refers to an end away from an operator during the surgical procedure, and "proximal end" refers to an end close to the operator during the surgical procedure.

### Embodiment 1

Referring to Fig. 3, this embodiment provides an ablation apparatus 20. The ablation apparatus 20 is configured to release ablation energy to a target tissue to substantially change an electrical shape, mechanical properties, chemical properties, or other properties of the target tissue. For ease of understanding, the embodiment is described with pulmonary nerve ablation as an application environment, but the application of the ablation apparatus 20 of the present disclosure is not limited thereto. The ablation apparatus may be used in a variety of different environments, such as ablation of tissues such as renal arteries, pulmonary veins, left atrial appendage, and ventricles.

The ablation apparatus 20 of this embodiment includes a catheter 21, an ablation portion 22, and an operation handle 23. The catheter 21 may be of a tubular structure, a proximal end of the catheter 21 is connected to the operation handle 23, and a distal end of the catheter 21 is connected to the ablation portion 22. The operation handle 23 is provided with a circuit connector (not shown in the figure) configured to connect to an energy generator (not shown in the figure). The energy generator is configured to generate ablation energy including, but not limited to, thermal energy, cold energy, electrical energy, acoustic energy, radio frequency (RF) energy, pulsed high voltage energy, mechanical energy, ionizing radiation, optical energy, combinations thereof, and other types of energy suitable for treating tissues. In the embodiment, the energy generator is an RF generator that may output the RF energy at a desired frequency, and in other embodiments, any other suitable type of energy generator may be selected. The ablation portion 22 has an expansible property and may be radially compressed under the action of an external force. After the external force is removed, the ablation portion 22 expands and returns to a naturally expanded state (i.e., a naturally deployed state without the action of an artificial external force). When performing an ablation operation, the above-mentioned catheter 21 and the ablation portion 22 may be accommodated in a delivery sheath (not shown in the figure) and delivered into an airway through the delivery sheath, and then the delivery sheath is withdrawn to release the ablation portion 22 so that the ablation portion 22 is expanded and deployed in the airway. The ablation portion 22 is controlled to output the ablation energy through the energy generator to ablate parasympathetic nerves in the bronchial adventitia, thereby blocking the transmission of nerve signals. After the ablation is completed, the output of the ablation energy is controlled to stop by operating the energy generator, and the catheter 21 and the ablation portion 22 are withdrawn into the delivery sheath and then withdrawn from the body together with the delivery sheath.

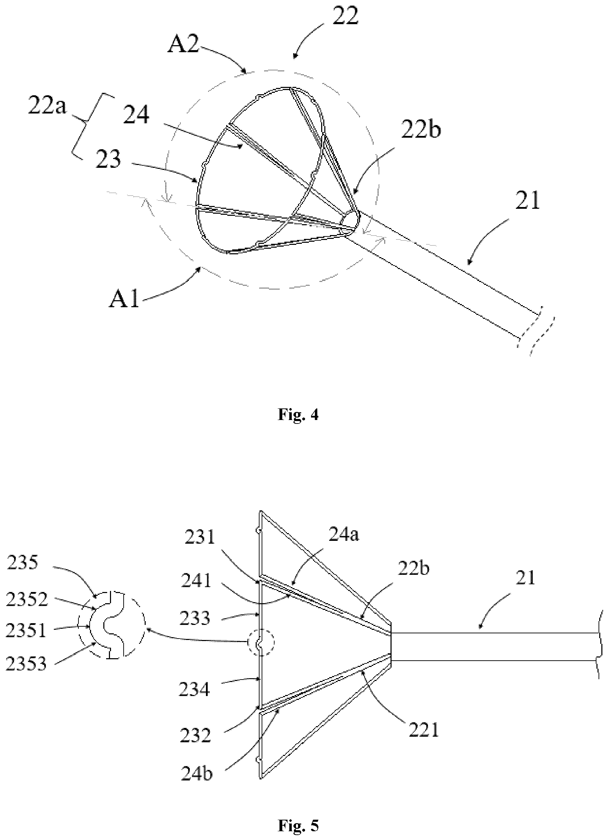

Referring to Fig. 4, it is a schematic structural diagram of the ablation portion 22 of this embodiment in a naturally expanded state. In the naturally expanded state, the ablation portion 22 includes ablation members 22a and supporting members 22b. A proximal end of the supporting member 22b is connected to the distal end of the catheter 21, and a distal end of the supporting member 22b is connected to the ablation member 22a. The ablation portion 22 has a first region A1 and a second region A2. The first region A1 is configured to attach to and ablate a relatively flat dorsal muscular wall portion 12 of the airway, and the second region A2 is configured to attach to and ablate a C-shaped region of the airway. For example, the ablation portion 22 of the embodiment includes six ablation members 22a in total. Two ablation members 22a are located in the first region A1, and the other four ablation members 22a are located in the second region A2. In other embodiments, the ablation portion 22 includes three ablation members 22a in total. One ablation member 22a is located in the first region A1, and the other two ablation members 22a are located in the second region A2. Alternatively, the ablation portion 22 includes four ablation members 22a in total. One ablation member 22a is located in the first region A1, and the other three ablation members 22a are located in the second region A2. A specific number of ablation members 22a may be selected based on the actual application environment, and the present disclosure is not limited thereto.

In this embodiment, the plurality of ablation members 22a of the ablation portion 22 are radially provided toward a distal end of the catheter 21 at intervals to form a conical structure. Referring also to Fig. 5, each ablation member 22a includes a supporting unit 24, a wall attachment unit 23, and an energy release unit provided on the wall attachment unit 23. Proximal ends of the supporting units 24 of the plurality of ablation members 22a are connected to the catheter 21 through the supporting members 22b and extend toward a distal end of the ablation portion 22 to be connected to the plurality of wall attachment units 23.

For example, the supporting units 24 of the six ablation members 22a are radially deployed outwardly and enclosed to form a cavity having an opening at a distal end. The supporting unit 24 of each ablation member 22a includes two first supporting rods 241 provided at spaced intervals, and distal ends of the two first supporting rods 241 are fixedly connected to the wall attachment unit 23 of the ablation member 22a by welding, bonding, etc. An included angle between each first supporting rod 241 and an axis of the ablation portion 22 (i.e., an angle inclined outwardly) is substantially equal, with a range of 25°-75°. In other embodiments, a number of first supporting rods 241 in the supporting unit 24 of each ablation member 22a may not be limited to two, may be one or more than two, and the number of first supporting rods 241 in the supporting unit 24 may be different in different ablation members 22a. The supporting unit 24 of the embodiment not only provides stable support for the wall attachment unit 23 in an axial direction, and reduces the displacement of the wall attachment unit 23 in the axial direction after the ablation portion 22 is deployed, but also provides a force to the wall attachment unit 23 toward an outer side of the ablation portion 22 (toward the outer side of the ablation portion 22 refers to a direction radially outward away from the central axis of the ablation portion 22, and toward an inner side of the ablation portion 22 refers to a direction radially inward close to the central axis of the ablation portion 22, the same below) so that the wall attachment unit 23 may be attached to an inner wall of a target tissue (e.g., airway) tightly to perform an ablation operation.

The supporting member 22b of this embodiment includes a plurality of second supporting rods 221. A proximal end of the second supporting rod 221 is fixedly connected to the distal end of the catheter 21, and a distal end of the second supporting rod 221 is fixedly connected to a proximal end of the first supporting rod 241. For example, the supporting member 22b of the embodiment includes six second supporting rods 221. Included angles between the six second supporting rods 221 and the axis of the ablation portion 22 (i.e., an angle inclined outwardly) are substantially equal, with a range of 25°-75°. Each second supporting rod 221 is connected to the proximal ends of two first supporting rods 241 adjacent to each other in two adjacent ablation members 22a. Two first supporting rods 241 connected to the same second supporting rod 221 may extend in parallel or non-parallel in different directions. Regardless of the extending direction of the two first supporting rods 241, it is necessary to ensure that there is a gap between the distal ends of the two first supporting rods 241 so that there is a certain activity space between the distal ends of the two first supporting rods 241. The second supporting rod 221 of this embodiment is provided to improve the supporting strength of the supporting unit 24. In addition, in the process of loading the ablation portion 22 into the delivery sheath, the ablation portion 22 located outside a distal port of the delivery sheath is driven to move toward the proximal end by pulling the catheter 21 located inside the delivery sheath toward a proximal direction, and the distal port of the delivery sheath exerts a radial compressive force on the ablation portion 22, thereby compressing the ablation portion 22. In the process, the second supporting rod 221 may guide the first supporting rod 241 to fold regularly, thereby making the ablation portion 22 easier to be radially compressed. It can be understood that, in other embodiments, the supporting member 22b may be of a mesh structure formed by cutting or weaving, and the mesh structure is tubular. Alternatively, the supporting member 22b may be a tubular structure or the like formed by axially arranging a plurality of interconnected wave coils. In other embodiments, the supporting member 22b may be omitted, and the proximal end of the supporting unit 24 of the ablation member 22a may be directly fixedly connected to the distal end of the catheter 21.

In the embodiment, to ensure that the supporting unit 24 and the supporting member 22b preferably maintain their natural expanded configuration, the supporting unit 24 and the supporting member 22b may be made of an elastic metal material by heat-setting. The elastic metal material includes known materials implanted in medical devices or a combination of various biocompatible materials, such as an alloy of two or more single metals of cobalt, chromium, nickel, titanium, magnesium, iron, and 316L stainless steel, nickel-titanium-tantalum alloy, or other elastic metal materials having biocompatibility. For example, the supporting unit 24 and the supporting member 22b of the embodiment are made of nickel-titanium alloy, and in other embodiments, the supporting unit 24 and the supporting member 22b may be made of different materials.

In this embodiment, each ablation member 22a includes one wall attachment unit 23. The wall attachment units 23 of the plurality of ablation members 22a are substantially located on a same radial plane (a plane perpendicular to the axis) and are provided around the above-mentioned opening enclosed by the plurality of supporting units 24. Each wall attachment unit 23 extends along an edge of the opening to form a substantially annular structure, configured to attach to the inner wall of the target tissue (e.g., airway).

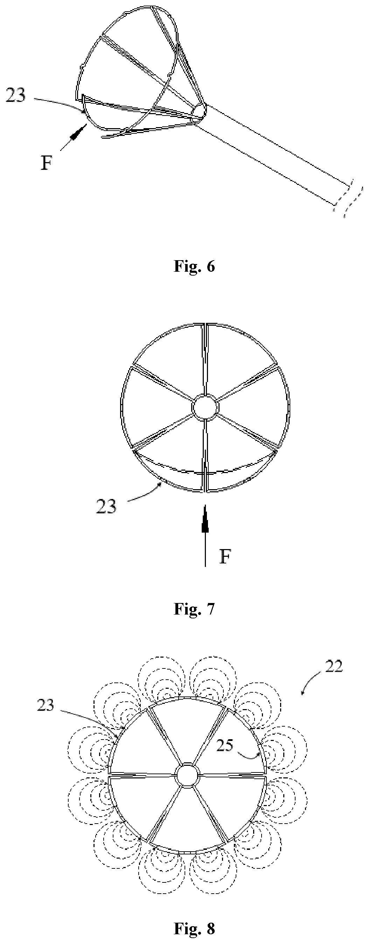

Referring to Figs. 5 and 6 together, the plurality of wall attachment units 23 of the embodiment have substantially the same shape, structure, and size and are provided at spaced intervals. Each wall attachment unit 23 may be a deformable unit and is independently deformed relative to the adjacent wall attachment units 23 when subjected to a force (e.g., a force F toward the inner side of the ablation portion 22). In other embodiments, the wall attachment unit 23 on the dorsal muscular wall portion 12 may be the deformable unit, and the other wall attachment units 23 are non-independently deformable wall attachment units 23. The specific shape, structure, and size of each wall attachment unit 23 may be selected as desired.

For example, the wall attachment unit 23 may be an elongated member and includes a first connecting end 231, a second connecting end 232, and a deformable section extending between the first connecting end 231 and the second connecting end 232. The first connecting end 231 and the second connecting end 232 are connected to two adjacent supporting units 24, respectively. For example, the first connecting end 231 is connected to the distal end of one of the first supporting rods 241 of a first supporting unit 24a, and the second connecting end 232 is connected to the distal end of one of the first supporting rods 241 of a second supporting unit 24b. In the embodiment, the above-mentioned first connecting end 231 and the second connecting end 232 are a head end and a tail end of the wall attachment unit 23. In other embodiments, the above-mentioned first connecting end 231 and the second connecting end 232 may be located in any position between the head end and the tail end of the wall attachment unit 23. In the embodiment, the first connecting end 231 and the second connecting end 232 of the deformable section are both connected to the supporting unit 24. When the ablation portion 22 is deployed in the airway, due to the outward expansion property, the supporting unit 24 may drive the first connecting end 231 and the second connecting end 232 on the wall attachment unit 23 to abut against the inner wall of the airway so that the deformable section located between the first connecting end 231 and the second connecting end 232 contacts the inner wall of the airway as much as possible. When the deformable section is squeezed by the inner wall of the airway, the deformable section is prevented from moving toward the inner side as a whole so that part of the region cannot contact the inner wall of the airway.

The deformable section is bent and protruded toward the outer side of the ablation portion 22 relative to the first connecting end 231 and the second connecting end 232 and has a deformable property to be elastically deformed when subjected to an external force. To ensure that the wall attachment unit 23 has a good elastic deformation property, the deformable section of the embodiment is made of nickel-titanium alloy. In other embodiments, the deformable section may be made of one or more of the above-mentioned elastic metal materials by heat-setting. Since the deformable section is bent and protruded toward the outer side of the ablation portion 22 and has an elastic deformation property, when the first connecting end 231 and the second connecting end 232 are driven by the supporting unit 24 to abut against the inner wall of the airway, the deformable section may be squeezed by the dorsal muscular wall portion 12 and deformed into a relatively flat shape to closely attach to an inner wall of the dorsal muscular wall portion 12.

For example, the deformable section includes a first wall attachment section 233 and a second wall attachment section 234. One end of the first wall attachment section 233 is connected to the first connecting end 231, the other end of the first wall attachment section 233 is connected to one end of the second wall attachment section 234, and the other end of the second wall attachment section 234 is connected to the second connecting end 232. The first wall attachment section 233 includes a first arcuate structure protruding toward the outer side of the ablation portion 22, and the second wall attachment section 234 includes a second arcuate structure protruding toward the outer side of the ablation portion 22. In other embodiments, the first wall attachment section 233 and the second wall attachment section 234 may also include a straight-line structure inclined toward the outer side of the ablation portion 22. The first wall attachment section 233 and the second wall attachment section 234 form an included angle, having a range of 175°-185°.

In order to facilitate folding of the deformable section into the sheath (i.e., accommodating into the delivery sheath), a connecting section 235 may be provided between the first wall attachment section 233 and the second wall attachment section 234. For example, the connecting section 235 includes a distal vertex 2351, and a first connecting arm 2352 and a second connecting arm 2353 jointly connecting the distal vertex 2351. A gap exists between a proximal end of the first connecting arm 2352 and a proximal end of the second connecting arm 2353. One end of the first wall attachment section 233 is connected to the first connecting end 231, and the other end of the first wall attachment section 233 is connected to the first connecting arm 2352. One end of the second wall attachment section 234 is connected to the second connecting arm 2353, and the other end of the second wall attachment section 234 is connected to the second connecting end 231. For example, the distal vertex 2351, the first connecting arm 2352, and the second connecting arm 2353 form a third arcuate structure. The third arcuate structure may be made of an elastic metal material, and the curvature of the third arcuate structure is greater than the curvature of the first arcuate structure and the curvature of the second arcuate structure. In the process of entering the sheath, the first connecting end 231 and the second connecting end 232 move close to each other by an opposite squeezing force, and the first wall attachment section 233 and the second wall attachment section 234 are folded toward each other. The third arcuate structure with a larger curvature is bent more so that the wall attachment unit 23 is more easily folded. In other embodiments, the distal vertex 2351, the first connecting arm 2352, and the second connecting arm 2353 described above may also form an inverted "V" shaped structure. The included angle of the inverted "V" shaped structure is less than the included angle between the first wall attachment section 233 and the second wall attachment section 234, for example, less than or equal to 175°. It should be noted that the connecting section 235 of this embodiment protrudes toward the distal end. The "toward the distal end" as used herein is not limited to being parallel to the central axis of the catheter 21, but may be inclined to the inner side or outer side of the ablation portion 22 to some extent, as long as the direction in which the connecting section 235 is protruded is directed toward the distal end. The connecting section 235 protruding toward the distal end not only facilitates guiding the wall attachment unit 23 to fold into a straight structure extending along the distal end, but also prevents a tip of the protrusion from contacting the inner wall of the airway after the ablation portion 22 is deployed, thereby effectively reducing damage and stimulation of the connecting section 235 that may be caused to the inner wall of the airway. It can be understood that, in other embodiments, the connecting section 235 may be protruded toward other directions, and the present disclosure is not limited thereto.

Referring again to Fig. 2, there is still an unattached region between the ablation apparatus 20 of an annular structure and the "D" shaped inner wall of the airway: the "dead angle" region 14. To achieve ablation of the parasympathetic nerves corresponding to the "dead angle" region 14, it is necessary to increase the ablation energy released by other electrodes (not shown in Fig. 2) that attach to the inner wall of the airway so that the generated ablation energy field covers the "dead angle" region 14 to achieve ablation of the "dead angle" region 14. However, as the ablation energy released by electrodes 25 increases, the large generated ablation energy field tends to cover an esophagus 13 near the dorsal muscular wall portion 12, thereby causing irreversible damage to the esophagus 13.

Referring to Figs. 7 and 8, in comparison with the annular structure 1 of Fig. 2, the wall attachment unit 23 of the embodiment is independently deformed to form a relatively straight structure when squeezed by the dorsal muscular wall portion 12 to closely attach to the dorsal muscular wall portion 12 so that the electrodes 25 on the wall attachment unit 23 release uniform ablation energy to the dorsal muscular wall portion 12, thereby achieving ablation of the target tissue while avoiding damage to the epithelial tissue of the dorsal muscular wall portion 12 and the adjacent esophagus 13.

In this embodiment, the energy release unit is configured to be connected to the energy generator and to apply ablation energy outputted by the energy generator to the target tissue. The energy release unit includes a plurality of electrodes 25. The electrode 25 may include, but is not limited to, one or more of a monopolar electrode, a bipolar electrode, a metal electrode, a strip electrode, and a needle electrode, or any other suitable electrode type. The arrangement and number of electrodes 25 may be determined by an electrode type, an electrode size, a range of the desired ablation region, etc. After a single ablation, the overlapping of ablation regions caused by the electrodes 25 may form a closed loop in a circumferential direction, thereby maximally blocking the transmission of pulmonary nerve signals.

For example, the ablation portion 22 is provided with 12 electrodes 25 in total, with each being the monopolar electrode. Each wall attachment unit 23 is provided with two electrodes 25, which are provided on the first wall attachment section 233 and the second wall attachment section 234, respectively. The electrodes 25 are arranged at uniform intervals to ensure that the ablation apparatus 20 of the embodiment forms uniform and continuous annular ablation areas at a certain depth of the target tissue, thereby maximally blocking the transmission of nerve signals. The electrode 25 is of a substantially tubular shape and is sheathed on an outer surface of the wall attachment unit 23. An insulating layer may also be provided between the electrode 25 and the wall attachment unit 23, for example, a polymer material having insulating properties such as Pebax, PTFE, and parylene. The insulating layer is provided to effectively prevent the conductivity of the wall attachment unit 23 from interfering with the RF and feedback circuits.

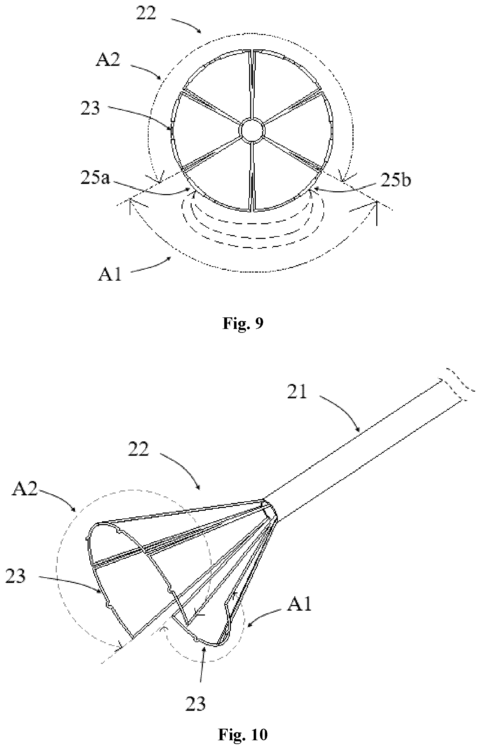

Referring to Fig. 9, in other embodiments, at least one electrode pair is included in the first region A1 of the ablation portion 22. The electrode pair includes a first electrode 25a and a second electrode 25b. Polarities of the first electrode 25a and the second electrode 25b are opposite. For example, the first electrode 25a is a positive electrode, and the second electrode 25b is a negative electrode. The first electrode 25a and the second electrode 25b are both electrically connected to the energy generator. In the first region A1, the first electrode 25a and the second electrode 25b are provided on two adjacent wall attachment units 23, respectively. When the energy generator outputs ablation energy to the first electrode 25a and the second electrode 25b, a relatively flat ablation energy field (a region marked by a dotted line in Fig. 9) is generated between the first electrode 25a and the second electrode 25b, and a relatively long and shallow damaged region is correspondingly generated. In comparison with the narrow and deep damaged region (a region marked by a dotted line in Fig. 8) generated by the monopolar electrode, the electrode 25 pair of the first region A1 of the embodiment may achieve ablation of the dorsal muscular wall portion 12 while avoiding damage to the epithelial tissue of the dorsal muscular wall portion 12 and the adjacent esophagus 13. In other embodiments, the above-mentioned electrode 25 pair may be replaced by a strip electrode. The strip electrode may be made of a metal material in a long strip shape and extend along a length direction of the wall attachment unit 23. The strip electrode may also generate a relatively flat ablation energy field, thereby avoiding damage to the epithelial tissue of the dorsal muscular wall portion 12 and the adjacent esophagus 13.

A sensor may also be provided in the electrode 25 to control the energy output and ablation effect during the ablation process. The sensor may be placed in the attachment of the electrode 25 and an airway wall to provide as accurate feedback as possible of the energy change in the ablation area. According to actual needs, the sensor may collect parameters such as temperature, pressure, and impedance. In other embodiments, the sensor may be omitted.

The ablation apparatus 20 may also include leads, and surfaces of the leads are provided with insulating layers. The leads may be connected inside the electrodes 25 by welding or the like, and a corresponding lead may be connected on the sensor as well. In the embodiment, the wall attachment unit 23, the supporting unit 24, the supporting member 22b, and the catheter 21 are all provided with internal passages communicating with each other, and the leads of the electrodes 25 and the sensor may be introduced into the internal passage of the wall attachment unit 23 together through corresponding through holes on the wall attachment unit 23. The leads of the plurality of electrodes 25 and the leads of the sensors may be collected inside the wall attachment unit 23, then pass through the internal passage of the wall attachment unit 23 and the internal passages of the supporting unit 24, a supporting structure, and the catheter 21 to reach the operation handle 23, and be communicated with the circuit connector. The internal passages in the wall attachment unit 23, the supporting unit 24, the supporting structure, and the catheter 21 of the embodiment have a restraining and protecting effect on the leads. In other embodiments, the internal passage of any one of the wall attachment unit 23, the supporting unit 24, and the catheter 21 may be omitted, and the leads may extend along surfaces of the wall attachment unit 23, the supporting unit 24, the supporting structure, and the catheter 21 as well and be communicated with the circuit connector in the operation handle 23.

### Embodiment 2

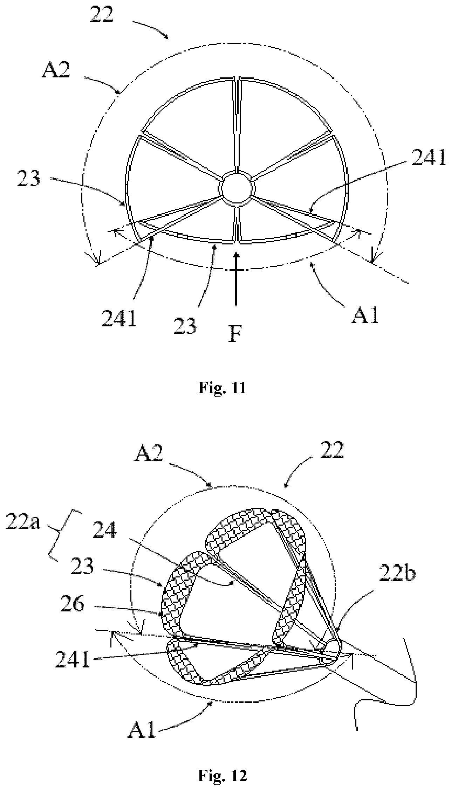

Referring to Figs. 10 and 11, this embodiment is substantially the same as the ablation apparatus 20 of embodiment 1, and the similarities will not be repeated, but the differences are that, the wall attachment units 23 in the first region A1 and the wall attachment units 23 in the second region A2 are located in different planes, and when both the first region A1 and the second region A2 are subjected to a force toward the inside of the ablation portion 22 (e.g., a radially inward force F), at least a part of the second region A2 may abut against an inner side of the wall attachment unit 23 in the first region A1 to apply an outward force to the wall attachment unit 23 in the first region A1. For example, when the ablation portion 22 of the embodiment is in the naturally expanded state, shapes and sizes of the wall attachment unit 23 in the first region A1 and the wall attachment unit 23 in the second region A2 are substantially the same, and the wall attachment unit 23 in the first region A1 is located between the wall attachment unit 23 in the second region A2 and the catheter 21, i.e., the axial distance from the wall attachment unit 23 in the first region A1 to the catheter 21 is less than the axial distance from the wall attachment unit 23 in the second region A2 to the catheter 21. Further, the included angle between the first supporting rod 241 of the first region A1 and the axis of the ablation portion 22 is greater than the included angle between the first supporting rod 241 of the second region A2 and the axis of the ablation portion 22. When the ablation portion 22 of the embodiment is deployed in the airway, both the first region A1 and the second region A2 are subjected to the radially inward squeezing force generated thereon by the inner wall of the airway, and the first region A1 and the second region A2 move close to each other under the action of this squeezing force so that the first supporting rods 241 at two side edges in the circumferential direction in the second region A2 abut against inner sides of the wall attachment unit 23 in the first region A1, thereby generating a radially outward abutting force on the wall attachment units 23 in the first region A1. The abutting force helps the wall attachment units 23 in the first region A1 to be deformed into a relatively flat shape, thereby attaching well to the dorsal muscular wall portion 12.

Due to the presence of the above-mentioned abutting force, the number of the first supporting rods 241 in the first region A1 may be appropriately reduced. For example, the first supporting rods 241 at the two side edges in the circumferential direction in the first region A1 may be omitted. The wall attachment units 23 in the first region A1 may also attach properly to the dorsal muscular wall portion 12, and due to the reduced number of the first supporting rods 241, it is advantageous to reduce the size of the ablation portion 22 after the radial compression so that the ablation portion 22 can easily enter the sheath.

### Embodiment 3

This embodiment is substantially the same as the ablation apparatus 20 of embodiment 1, and the similarities will not be repeated, but the differences are that specific structures of the wall attachment units 23 are different. Referring to Fig. 12, the wall attachment unit 23 of the embodiment include a mesh structure 26. In the embodiment, the wall attachment units 23 in the first region A1 configured to attach to the relatively flat dorsal muscular wall portion 12 in the airway each have a mesh structure 26, and the wall attachment units 23 in the second region A2 configured to attach to the C-shaped region of the airway each also have a mesh structure 26. In other embodiments, only the wall attachment unit 23 in the first region A1 includes the mesh structure 26, and for a structure of the wall attachment unit 23 in the second region A2, please refer to embodiment 1, or only the wall attachment unit 23 in the second region A2 includes the mesh structure 26, and for a structure of the wall attachment unit 23 in the first region A1, please refer to embodiment 1.

In this embodiment, the mesh structure 26 has a sheet shape and may be formed by cutting or weaving. For example, the mesh structure 26 includes a plurality of first direction supporting wires arranged at spaced intervals and a plurality of second direction supporting wires arranged at spaced intervals. The first direction supporting wire extends substantially along a first direction and the second direction supporting wire extends substantially along a second direction. The first direction supporting wires and the second direction supporting wires are overlapped with each other to form a plurality of quadrangular meshes and a plurality of intersecting units. The intersecting unit includes an intersecting point formed by the first direction supporting wire and the second direction supporting wire. The quadrangular mesh is substantially square, but may also be diamond, rectangular, and other shapes. Four intersecting units are correspondingly provided at the positions of the four corners of the quadrangular mesh. An edge contour of the mesh structure 26 is arcuate and is bent and protrudes toward the outer side of the ablation portion 22. The mesh structure 26 has a deformable property and is elastically deformed when subjected to an external force. Adjacent mesh structures 26 have a gap at least at distal portions to block the transmission of force between the two mesh structures 26 so that the mesh structure 26 is independently deformed relative to adjacent mesh structures 26 to adapt to the "D" shaped cross-section of the inner wall of the airway.

Two ends of the above-mentioned mesh structure 26 are connected to the supporting unit 24 in the circumferential direction. For example, one end of the mesh structure 26 in the circumferential direction is fixedly connected to the distal end of one of the first supporting rods 241 of the first supporting unit 24a by bonding, welding, etc., and the other end of the mesh structure 26 in the circumferential direction is fixedly connected to the distal end of one of the first supporting rods 241 of the second supporting unit 24b. Since the mesh structures 26 are all connected to the supporting units 24, when the ablation portion 22 is deployed in the airway, the supporting units 24 may drive the mesh structures 26 to radially expand and abut against the inner wall of the airway, and when the mesh structures 26 are squeezed by the inner wall of the airway, the mesh structures 26 are deformed to conform to the shape of the inner wall of the airway, thereby closely attaching to the inner wall of the airway.

In this embodiment, the above-mentioned supporting wire is a conductive wire, and the conductive wire is exposed to form a mesh electrode. After the conductive wire is communicated with the energy generator and energized, it forms the mesh electrode to output RF energy to destroy nerves in the airway tissue. The conductive wire may be a nickel-titanium alloy wire, a stainless-steel wire, or any other wire that may be conductive. The above-mentioned supporting unit 24 may also be made of a conductive metal, and an outer surface is covered with an insulating layer. The supporting unit 24 is communicated with the mesh electrode and the energy generator, and the electric energy provided by the energy generator may be transmitted to the mesh electrode through the supporting unit 24 so that the mesh electrode forms an ablation energy field. Therefore, the mesh electrode does not need to be additionally provided with a lead.

The plurality of mesh structures 26 arranged in the circumferential direction may form a continuous annular ablation damaged area. Compared with the way in which a plurality of point electrodes 25 generate a plurality of overlapping annular energy fields, the energy of the mesh electrodes is more uniform and is easier to control the outputted ablation energy, reducing the probability of the ablation apparatus 20 causing burns to the airway.

Further, the first supporting wire and the second supporting wire of the above-mentioned mesh structure 26 may slide or be fixed to each other at the intersecting point. For the mesh structure 26 in which the first supporting wire and the second supporting wire may slide with each other at the intersection point, when squeezed by the airway wall, the quadrangular meshes in the mesh structure 26 are easily deformed so that the first supporting wires and the second supporting wires in some regions are accumulated, and the first supporting wires and the second supporting wires in some regions are relatively sparse. At this time, if both the first supporting wire and the second supporting wire are conductive wires, the ablation energy generated in some regions of the mesh electrode will be strong, while the ablation energy generated in some regions will be weak, resulting in incomplete ablation of the nerves in a part of the airway wall and damage to a part of the airway wall by excessive ablation energy. In order to prevent the above-mentioned problem, the first supporting wires and the second supporting wires at at least a part of the intersecting points in the mesh structure 26 are fixedly connected. For example, the first supporting wires and the second supporting wires at all intersecting points in the mesh structure 26 in the first region A1 are fixedly connected, such as by cutting to form the mesh structure 26 in the first region A1, or, for a woven mesh structure 26, the intersecting points are fixed by welding, bonding, winding, etc. The supporting wires in the mesh structure 26 thus formed are not prone to slippage with each other and are uniformly distributed even when subjected to squeezing forces, thereby forming a uniform ablation energy field that avoids damage to the dorsal muscular wall portion 12 and the airway wall.

In other embodiments, the wall attachment unit 23 may further include a plurality of waveform units arranged along the axial direction at intervals. The plurality of waveform units form the mesh structure 26, and each waveform unit includes a plurality of waves connected end-to-end and arranged in the circumferential direction.

### Embodiment 4

In the embodiment, based on embodiments 1-3, in order to reduce the possibility of irreversible damage to the target tissue (e.g., airway epithelial tissue) caused by heating during the ablation process, the epithelial tissue in a target region may be cooled during ablation.

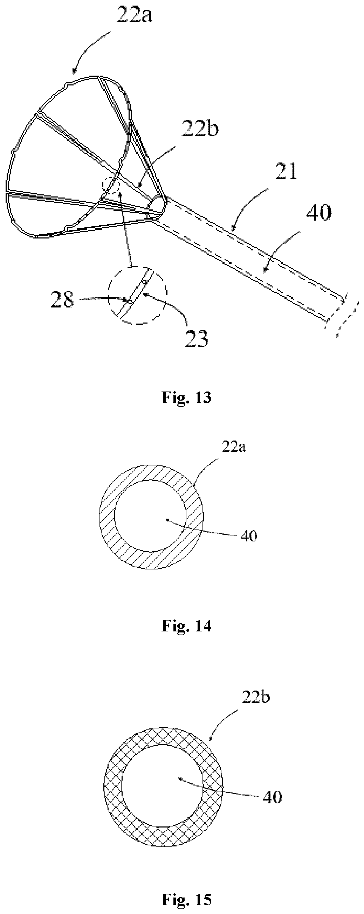

Referring to Figs. 13-15, the operation handle 23 (referring to Fig. 3) of the embodiment is further provided with an interface configured to connect a cooling apparatus (not shown in the figure). Interiors of the catheter 21, the supporting member 22b, and the ablation member 22a are all provided with delivery channels 40 that communicate with each other. The delivery channel 40 is further communicated with a connected cooling apparatus, and at least one first cooling hole 28 is arranged on the wall attachment unit 23 and/or the energy release unit. The first cooling hole 28 communicates the delivery channel 40 with the outside. The cooling apparatus may deliver a cooling medium (e.g., normal saline) to the delivery channel 40, and the cooling medium may then be discharged outwardly through the first cooling hole 28 to the target tissue (e.g., in direct contact with the epithelial tissue of the airway) to take away a part of the heat. The cooling medium discharged into the target tissue may be recovered through a bronchoscope after treatment.

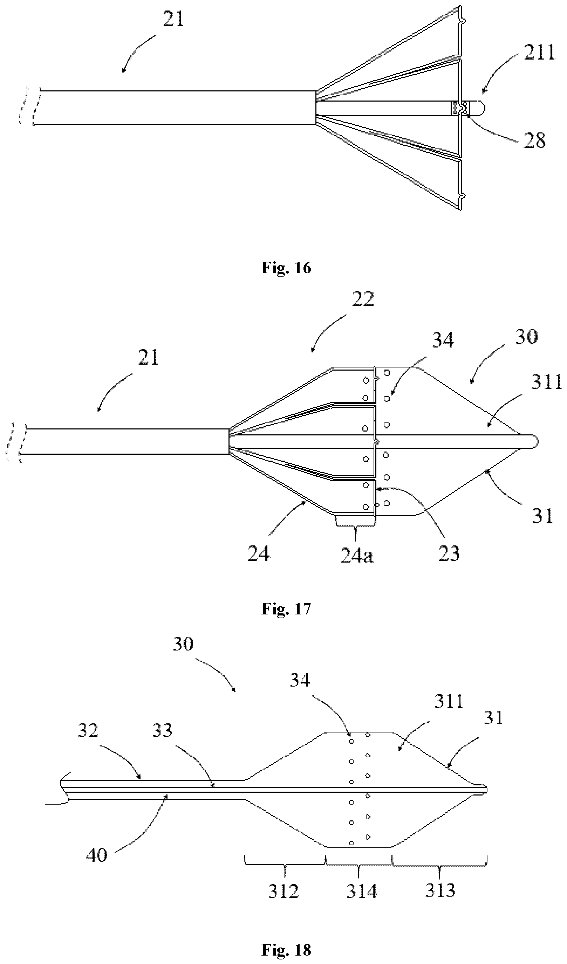

Referring to Fig. 16, in other embodiments, one extension tube 211 may be fixedly connected to the distal end of the catheter 21, and the extension tube 211 and the catheter 21 are provided with delivery channels 40 that communicate with each other. At least one first cooling hole 28 is arranged on a surface of the extension tube 211. The first cooling hole 28 communicates the delivery channel 40 with the outside and is substantially flush (substantially in the same radial plane) with at least one energy release unit. The cooling apparatus may deliver a cooling medium (e.g., normal saline) to the delivery channel 40, and the cooling medium may then be injected outwardly through the first cooling hole 28 to the target tissue (e.g., in direct contact with the epithelial tissue of the airway) to take away a part of the heat. In this process, pressure may be appropriately applied to the cooling medium to increase an injection range of the cooling medium. Due to the addition of the extension tube 211, the delivery channels 40 inside the supporting member 22b and the ablation member 22a may be omitted.

### Embodiment 5

The embodiment proposes another cooling treatment based on embodiment 4.

Referring to Figs. 17 and 18, the ablation apparatus 20 of the embodiment further includes a cooling balloon 30. The cooling balloon 30 includes a balloon main body 31 having a balloon chamber 311, an outer tube 32 connected to the balloon main body 31, an inner rod 33, and second cooling holes 34 arranged on a surface of the cooling balloon 30. The balloon main body 31 may be radially expanded and compressed. When the balloon main body 31 is in an expanded state, the balloon main body 31 includes a proximal section 312, a distal section 313, and a middle section 314 with two ends connected to the proximal section 312 and the distal section 313, respectively. The shape of a cross-section of the balloon main body 31 is substantially circular. In other embodiments, the cross-section of the balloon main body 31 may also be any other suitable shape, such as an oval shape, a crescent shape, and a semi-circle shape. The proximal section 312 and the distal section 313 are substantially tapered, and the middle segment 314 is substantially cylindrical. In the embodiment, the cross-sectional area of the proximal end of the proximal section 312 is less than the cross-sectional area of the distal end of the proximal section 312, cross-sectional areas of the middle section 314 are substantially equal, and the cross-sectional area of the proximal end of the distal section 313 is greater than the cross-sectional area of the distal end of the distal section 313. The balloon main body 31 may be a compliant balloon, a non-compliant balloon, or a semi-compliant balloon and may be made of one or a mixture of nylon, Pebax, polyurethane, polyethylene terephthalate, and polyethylene.

The outer tube 32 passes through the catheter 21, and the proximal end of the outer tube 32 is connected to the cooling apparatus through the operation handle 23. The distal end of the outer tube 32 is connected to the balloon main body 31. The inner rod 33 extends through a lumen of the outer tube 32, and the outer diameter of the inner rod 33 is less than the inner diameter of the outer tube 32. The proximal end of the inner rod 33 is connected to the operation handle 23, and the distal end of the inner rod 33 is located in the balloon main body 31 and is connected to the distal end of the balloon main body 31. A gap is formed between the outer tube 32 and the inner rod 33 as the delivery passage 40. The delivery channel 40 communicates with the balloon chamber 311 of the balloon main body 31 and is configured to deliver the cooling medium in the cooling apparatus into the balloon chamber 311. The balloon chamber 311 is in an expanded state when inflated by the cooling medium. When the cooling medium in the balloon chamber 311 is discharged, the balloon chamber 311 is shrunk, and at this time, the balloon main body 31 is in a shrinkage state.

In this embodiment, a circle of second cooling holes 34 is arranged on the surface of the balloon main body 31 and include a plurality of second cooling holes 34 arranged along the circumferential direction at intervals. In other embodiments, a plurality of circles of second cooling holes 34 may also be arranged on the surface of the balloon, or a completely different arrangement of the second cooling holes 34 than in the embodiment may be adopted.

In the process of ablation, the cooling balloon 30 in the shrinkage state is first inserted into the ablation portion 22, and the cooling apparatus delivers the cooling medium to the balloon chamber 311 of the balloon main body 31 through the delivery channel 40 so that the balloon main body 31 is in the expanded state. An outer surface of the expanded balloon main body 31 is tightly attached to the inner wall of the target tissue, and the cooling medium is discharged to the inner wall of the target tissue through the second cooling holes 34. It should be noted that since the electrode 25 outputs the ablation energy to heat the surrounding tissue, the second cooling holes 34 should be located as close to the electrode 25 as possible. For example, the plurality of second cooling holes 34 are located near the distal end surface of the ablation portion 22 so that the cooling medium is discharged to the heated tissue as much as possible to improve the cooling effect.

In the embodiment, the target tissue is cooled through the cooling balloon 30, effectively reducing the possibility of irreversible damage to the target tissue after heating. In addition, when the cooling balloon 30 is in the expanded state, it may also promote better wall attachment of the ablation portion 22.

Further, the shape of the ablation portion 22 used in conjunction with the cooling balloon 30 of the embodiment is slightly different from that of the ablation portions 22 of the embodiments 1-4, in that the supporting unit 24 of the ablation portion 22 used in conjunction with the cooling balloon 30 of this embodiment is bent at a distal end to form a straight section 24a and is fixedly connected with the wall attachment unit 23 through the straight section 24a. The included angle between the straight section 24a and the axis of the ablation portion 22 is less than the included angle between a remaining region of the supporting unit 24 and the axis of the ablation portion 22, and the included angle between the straight section 24a and the axis of the ablation portion 22 ranges from 0-10°. The presence of the straight section 24a not only improves the wall attachment effect of the ablation portion 22, but also increases an attachment region between the cooling balloon 30 and the target tissue.

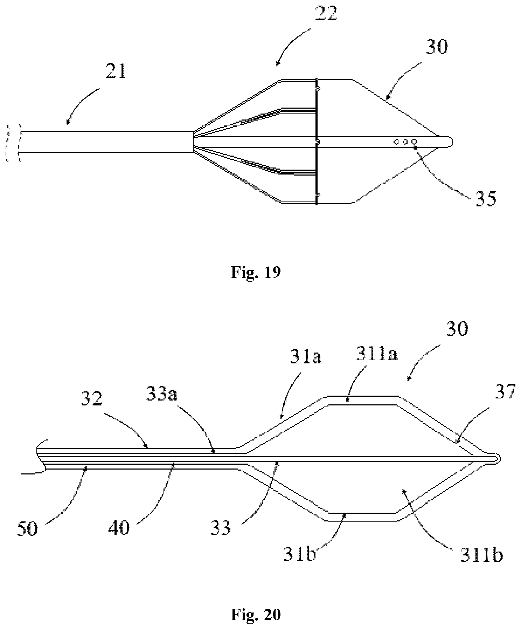

Referring to Fig. 19, in other embodiments, the above-mentioned second cooling hole 34 may be omitted, and a recovery channel 50 is formed in the inner rod 33. The proximal end of the recovery channel 50 connects a recovery apparatus through the operation handle 23 so that the recovery channel 50 communicates with the recovery apparatus. Recovery holes 35 that communicate with the recovery channel 50 and the balloon chamber 311 are arranged on the inner rod 33, and the cooling medium in the balloon chamber 311 is recovered through the recovery channel 50 by applying a negative pressure to the recovery channel 50, thereby constituting a cooling circuit. The construction of the cooling circuit facilitates continuous cooling of the target tissue and avoids excessive cooling medium entering the human body.

Further, the distance from the recover hole 35 to the distal end of the balloon main body 31 is less than the distance from the recover hole 35 to the proximal end of the balloon main body 31. This arrangement allows the cooling medium to be recovered after sufficient contact with the tissue in the ablation area to provide maximum cooling for the target tissue.

In other embodiments, the inner rod 33 may be located outside the outer tube 32 and the balloon main body 31 and may also constitute a cooling circuit together with the outer tube 32 and the balloon main body 31. In addition, in other embodiments, the inner rod 33 may be omitted, and the outer tube 32 may not only have the delivery channel 40 to deliver the cooling medium into the balloon main body 31, but may also have the recovery channel 50 to output the cooling medium in the balloon main body 31 to the recovery apparatus. The input and recovery of the cooling medium during ablation may be repeated multiple times according to the cooling requirements.

### Embodiment 6

The embodiment proposes another cooling balloon 30 based on embodiment 5.

Referring to Fig. 20, the cooling balloon 30 of the embodiment includes a first balloon main body 31a having a balloon chamber, an outer tube 32 fixedly connected to the proximal end of the first balloon main body 31a, a second balloon main body 31b, an inner tube 33a fixedly connected to the proximal end of the second balloon main body 31b, and an inner rod 33. The first balloon main body 31a has a first balloon chamber 311a, and the second balloon main body 31b has a second balloon chamber 311b. Specific structures of the two are substantially the same as those of embodiment 6. The first balloon main body 31a surrounds the outside of the second balloon main body 31b, and the first balloon main body 31a and the second balloon main body 31b are coaxially provided. A cooling cavity is formed between the inner surface of the first balloon main body 31a and the outer surface of the second balloon main body 31b. The inner tube 33a extends through the lumen of the outer tube 32, and the outer diameter of the inner tube 33a is less than the inner diameter of the outer tube 32. The proximal end of the inner tube 33a connects the cooling apparatus through the operation handle. The outer tube 32 passes through the catheter 21, and the proximal end of the outer tube 32 connects the recovery apparatus through the operation handle 23. The lumen of the inner tube 33a forms the delivery channel 40. The delivery channel 40 communicates with the second balloon chamber 311b and is configured to deliver the cooling medium in the cooling apparatus into the second balloon chamber 311b. A recovery channel 50 is formed between the outer surface of the inner tube 33a and the inner surface of the outer tube 32. The recovery channel 50 communicates with the first balloon chamber 311a and is configured to recover the cooling medium in the first balloon chamber 311a. One or more output holes 37 are arranged on the second balloon main body 31b. The output hole 37 communicates the first balloon chamber 311a and the second balloon chamber 311b and is configured to output the cooling medium in the second balloon main body 31b into the first balloon chamber 311a after the second balloon main body 31b is inflated with the cooling medium and expanded. After the first balloon main body 31a is inflated and expanded, the cooling medium in the first balloon chamber 311a may be discharged to the recovery apparatus via the recovery channel 50. The inner rod 33 passes through the inner tube 33a, the proximal end of the inner rod 33 is connected to the operation handle 23, and the distal end of the inner rod 33 passes through the second balloon chamber 311b and is fixedly connected to the second balloon main body 31b. The arrangement of inner rod 33 not only serves to reinforce the outer tube 32, but also provides support to the balloon main body 31 when the balloon main body 31 is in a compressed state, maintaining the stability of the shape of the balloon main body 31 and preventing the balloon main body 31 from shortening.

During the process of ablation, the cooling balloon 30 in the shrinkage state is first inserted into the ablation portion 22, and the cooling apparatus delivers the cooling medium to the second balloon chamber 311b through the delivery channel 40 so that the second balloon main body 31b is in the expanded state. The cooling medium in the second balloon main body 31b flows into the first balloon main body 31a via the output hole 37 so that an outer surface of the first balloon main body 31a is attached to the inner wall of the target tissue tightly to cool the inner wall of the target tissue. The cooling medium in the first balloon main body 31a is recovered through the recovery channel 50 by applying a negative pressure to the recovery channel 50, thereby constituting a cooling circuit. The construction of the cooling circuit facilitates continuous cooling of the target tissue and avoids excessive cooling medium entering the human body.

In this embodiment, the first balloon main body 31a and the second balloon main body 31b are coaxially provided, and a cooling cavity is formed between the first balloon main body 31a and the second balloon main body 31b so that the flow rate of the cooling medium in the cooling cavity may be faster, improving the cooling efficiency of the outer surface of the balloon main body 31.

Further, the distance from the output hole 37 to the distal end of the second balloon main body 31b is less than the distance from the output hole 37 to the proximal end of the second balloon main body 31b. This arrangement allows the cooling medium to be recovered after sufficient contact with the tissue in the ablation area to provide maximum cooling of the target tissue.

### Embodiment 7

The embodiment proposes another cooling balloon 30 based on embodiments 5 and 6.

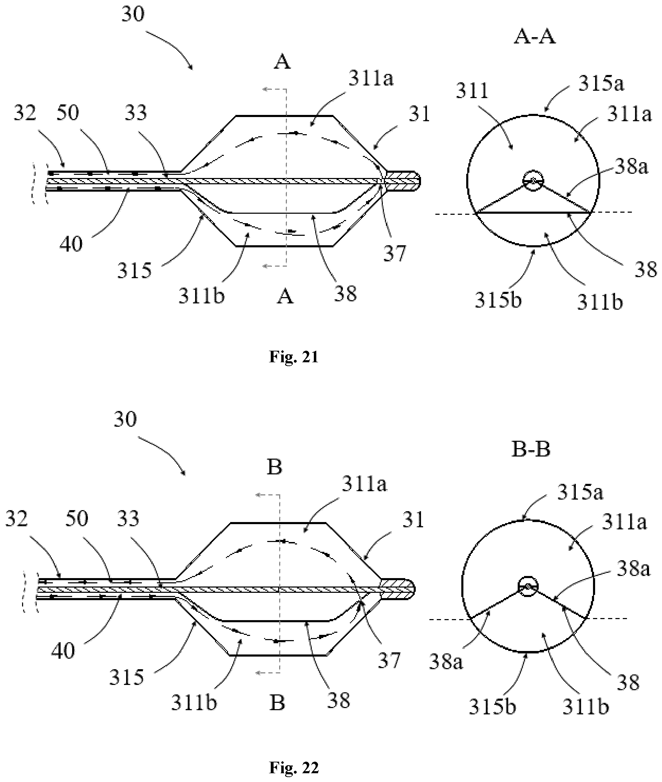

Referring to Fig. 21, the cooling balloon 30 of the embodiment includes an expandable balloon main body 31, and an outer tube 32 and an inner rod 33 fixedly connected to the balloon main body 31. The balloon main body 31 includes a balloon wall 315 and a balloon chamber 311 enclosed by the balloon wall 315. The balloon chamber 311 includes a first balloon chamber 311a, a second balloon chamber 311b, and a separating piece 38 which are radially arranged. The separating piece 38 is fixedly connected to an interior of the balloon main body 31 and is provided between the first balloon chamber 311a and the second balloon chamber 311b, configured to separate the balloon chamber 311 into the first balloon chamber 311a and the second balloon chamber 311b. The first balloon chamber 311a has a first outer wall 315a, and the second balloon chamber 311b has a second outer wall 315b. The first outer wall 315a and the second outer wall 315b are arranged in the circumferential direction of the balloon main body 31 and are configured to attach to the target tissue when the balloon main body 31 is expanded. The distal end of the outer tube 32 is connected to the proximal end of the balloon main body 31. The outer tube 32 is a multi-chamber tube, and its interior has an accommodating passage, a delivery channel 40, and a recovery channel 50 independent of each other. The accommodating passage is configured to accommodate the inner rod 33. The inner rod 33 extends through the accommodating passage, and the outer diameter of the inner rod 33 is less than or equal to the inner diameter of the accommodating passage. The proximal end of the inner rod 33 is connected to the operation handle 23. The distal end of the inner rod 33 is located in the balloon main body 31 and is connected to the distal end of the balloon main body 31. The arrangement of the inner rod 33 not only serves to reinforce the outer tube 32, but also provides support to the balloon main body 31 when the balloon main body 31 is in a compressed state, maintaining the stability of the shape of the balloon main body 31 and preventing the balloon main body 31 from shortening or curling and folding. The proximal end of the delivery channel 40 communicates with the connected cooling apparatus, and its distal end communicates with the second balloon chamber 311b for delivering the cooling medium in the cooling apparatus into the second balloon chamber 311b. The proximal end of the recovery channel 50 communicates with the connected recovery apparatus, and its distal end communicates with the first balloon chamber 311a for outputting the cooling medium from the first balloon chamber 311a to the recovery apparatus after the first balloon chamber 311a is inflated with the cooling medium and expanded.

At least one output hole 37 is arranged between the first balloon chamber 311a and the second balloon chamber 311b. The output hole 37 communicates the first balloon chamber 311a and the second balloon chamber 311b and is configured to flow the cooling medium between the first balloon chamber 311a and the second balloon chamber 311b. Referring to Fig. 21, the separating piece 38 includes a laminar structure 38a, its proximal end and distal edge are fixedly connected to the inner rod 33, and its two side edges are sealingly connected to the balloon wall 315. The output hole 37 may be arranged on the inner rod 33 between the distal edge of the separating piece 38 and the distal end of the balloon main body 31. Referring to Fig. 22, in other embodiments, the output hole 37 is arranged on the separating piece 38. In other embodiments, a gap may be left between the distal edge of the separating piece 38 and the distal end of the balloon main body 31, the gap communicates the first balloon chamber 311a and the second balloon chamber 311b and may serve as the output hole 37.

During the process of ablation, the cooling balloon 30 in the shrinkage state is first inserted into the ablation portion 22, and the cooling apparatus delivers the cooling medium into the second balloon chamber 311b through the delivery channel 40 so that the second balloon chamber 311b is in the expanded state, and the second outer wall 315b of the second balloon chamber 311b is attached to the target tissue. The cooling medium in the second balloon chamber 311b flows into the first balloon chamber 311a via the output hole 37 so that the first balloon chamber 311a is in the expanded state, and the first outer wall 315a of the first balloon chamber 311a is attached to the target tissue to cool the inner wall of the target tissue.

The cooling medium in the first balloon chamber 311a is recovered to the connected recovery apparatus through the recovery channel 50 by applying a negative pressure to the recovery channel 50, thereby constituting a cooling circuit. The construction of the cooling circuit facilitates continuous cooling of the target tissue and avoids excessive cooling medium entering the human body.

Further, the distance from the output hole 37 to the distal end of the balloon main body 31 is less than the distance from the output hole 37 to the proximal end of the balloon main body 31. This arrangement allows the cooling medium to be recovered after sufficient contact with the tissue in the ablation area to provide maximum cooling for the target tissue.

The relatively flat dorsal muscular wall portion 12 in the airway is more susceptible to scarring during ablation than the "C" shaped region in the airway, and in order to better protect the epithelial tissue of the dorsal muscular wall portion 12, the cooling efficiency of the cooling balloon 30 at the dorsal muscular wall portion 12 needs to be improved. However, the cooling efficiency (the speed of taking away the heat of the target tissue) of the cooling balloon 30 is closely related to the flow rate of the cooling medium. The faster the flow rate of the cooling medium, the faster the speed of taking away the heat, and the slower the flow rate of the cooling medium, the slower the speed of taking away the heat.

In this embodiment, the second outer wall 315b is configured to attach to the dorsal muscular wall portion 12, while the first outer wall 315a is configured to attach to the "C" shaped region of the airway. Any cross-sectional area of the first balloon chamber 311a in an axial region where the first outer wall 315a is located is denoted as a first cross-sectional area, and any cross-sectional area of the second balloon chamber 311b in an axial region where the second outer wall 315b is located is denoted as a second cross-sectional area. The first cross-sectional area is greater than the second cross-sectional area. When both the first balloon chamber 311a and the second balloon chamber 311b are in the expanded state, and the delivery speed of the cooling medium in the delivery channel 40 (i.e., an input speed of the cooling medium in the balloon main body 31) is the same as a recovery speed of the cooling medium in the recovery channel 50 (i.e., an output speed of the cooling medium in the balloon main body 31), the communicating first balloon chamber 311a and the second can expand to form a fluid channel, and the flow of the cooling medium of each cross-section in the fluid channel is consistent. However, the smaller second cross-sectional area makes the flow area of the cooling medium in the axial region where the second outer wall 315b is located in the second balloon inner chamber 311b smaller than the flow area of the cooling medium in the axial region where the first outer wall 315a is located in the first balloon inner chamber 311a, and since the flow of a certain cross-section is the product of the flow rate and the flow area (i.e., the cross-sectional area) of the cross-section, the flow rate of the cooling medium in the axial region where the second outer wall 315b is located is greater than the flow rate of the cooling medium in the axial region where the first outer wall 315a is located. Further, the cooling efficiency of the cooling balloon 30 at the second outer wall 315b is higher than the cooling efficiency of the cooling balloon 30 at the first outer wall 315a.

For example, referring to Fig. 21, the axial regions where the first outer wall 315a and the second outer wall 315b of the cooling balloon 30 are located overlap, and the separating piece 38 includes a single laminar structure 38a. Fig. 21 shows a schematic diagram of a cross-section of the balloon main body 31 within the axial regions where the first outer wall 315a and the second outer wall 315b are located. The shape of the cross-section of the balloon main body 31 is generally circular, and the shape of the cross-section of the separating piece 38 is a straight line. The separating piece 38 separates the cross-section of the balloon main body 31 into two half-moon shaped cross-sections, i.e., a first cross-section and a second cross-section, respectively. The first cross-section is the cross-section of the first balloon chamber 311a, the second cross-section is the cross-section of the second balloon chamber 311b, and the ratio of the area of the first cross-section to the area of the second cross-section ranges from R1, R1∈(1,6]. In other embodiments, the balloon main body 31 may have a cross-sectional area occupied by the first balloon chamber 311a greater than a cross-sectional area occupied by the second balloon chamber 311b in any cross-section of the balloon main body 31 in the axial region where the separating piece 38 is located.

Referring to Fig. 22, in another embodiment, the axial regions where the first outer wall 315a and the second outer wall 315b of the cooling balloon 30 are located overlap, and the separating piece 38 includes two laminar structures 38a. Each laminar structure 38a has one side edge sealingly connected to the inner rod 33 and an opposite side edge sealingly connected to the balloon wall 315 to separate the balloon chamber 311 into the first balloon chamber 311a and the second balloon chamber 311b. Fig. 22 shows a schematic diagram of a cross-section of the balloon main body 31 within the axial regions where the first outer wall 315a and the second outer wall 315b are located. The shape of the cross-section of the balloon main body 31 is generally circular, and the shape of the cross-section of the separating piece 38 is a folded line (inverted "V" shape). The separating piece 38 separates the cross-section of the balloon main body 31 into two fan-shaped cross-sections, i.e., a first cross-section and a second cross-section, respectively. The ratio of the central angle of the first cross-section to the central angle of the second cross-section ranges from R2, R2∈(1,3]. The first cross-section is the cross-section of the first balloon chamber 311a, the second cross-section is the cross-section of the second balloon chamber 311b, and the area of the first cross-section is greater than the area of the second cross-section.

Further, the flow rate of the cooling medium in the first balloon chamber 311a and the second balloon chamber 311b may also be controlled by adjusting the surface roughness of inner surfaces of the first outer wall 315a and the second outer wall 315b. For example, the surface roughness of the inner surface of the second outer wall 315b is made smaller than the surface roughness of the inner surface of the first outer wall 315a so that the flow rate of the cooling medium flowing through the inner surface of the second outer wall 315b is greater than the flow rate of the cooling medium flowing through the inner surface of the first outer wall 315a. In other embodiments, the inner surface roughness of the first chamber wall enclosing the first balloon chamber 311a may also be made greater than the inner surface roughness of the second outer wall 315b enclosing the second balloon chamber 311b.

Further, the first outer wall 315a and the second outer wall 315b may be made of different materials so that the outer surface hardness of the second outer wall 325b is less than the outer surface hardness of the first outer wall 315a, thereby allowing the second outer wall 325b to more closely attach to the dorsal muscular wall portion 12.

### Embodiment 8

The embodiment proposes another cooling balloon 30 based on embodiments 5-7.

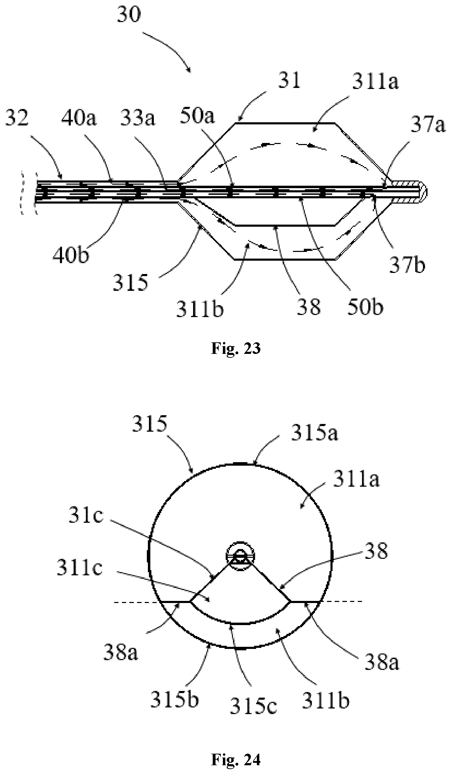

Referring to Fig. 23, the cooling balloon 30 of the embodiment includes an expandable balloon main body 31, and an outer tube 32 and an inner tube 33a fixedly connected to the balloon main body 31. The balloon main body 31 includes a balloon wall 315 and a balloon chamber 311 enclosed by the balloon wall 315. The balloon chamber 311 includes a first balloon chamber 311a, a second balloon chamber 311b, and a separating piece 38 which are radially arranged. The separating piece 38 is fixedly connected to an interior of the balloon main body 31 and is provided between the first balloon chamber 311a and the second balloon chamber 311b, configured to separate the balloon chamber 311 into the first balloon chamber 311a and the second balloon chamber 311b. The first balloon chamber 311a has a first outer wall 315a, and the second balloon chamber 311b has a second outer wall 315b. The first outer wall 315a and the second outer wall 315b are arranged in the circumferential direction of the balloon main body 31 and are configured to attach to the target tissue when the balloon main body 31 is expanded. The distal end of the outer tube 32 is connected to the proximal end of the balloon main body 31, and the proximal end of the outer tube 32 connects the cooling apparatus through the operation handle 23. The inner tube 33a extends through the lumen of the outer tube 32, and the outer diameter of the inner tube 33a is smaller than the inner diameter of the outer tube 32. The proximal end of the inner tube 33a connects the recovery apparatus through the operation handle 23, and the distal end of the inner tube 33a is located in the balloon main body 31 and is connected to the distal end of the balloon main body 31. The outer tube 32 is a multi-chamber tube, and its interior has an accommodating passage, a first delivery channel 40a, and a second delivery channel 40b independent of each other. The accommodating passage is configured to accommodate the inner tube 33a, and proximal ends of the first delivery channel 40a and the second delivery channel 40b communicate with the connected cooling apparatus. The distal end of the first delivery channel 40a communicates with the first balloon chamber 311a for delivering the cooling medium in the cooling apparatus into the first balloon chamber 311a. The distal end of the second delivery channel 40b communicates with the second balloon chamber 311b for delivering the cooling medium in the cooling apparatus into the second balloon chamber 311b. The inner tube 33a is also a multi-chamber tube, and its interior has a first recovery channel 50a and a second recovery channel 50b independent of each other. Proximal ends of the first recovery channel 50a and the second recovery channel 50b communicate with the connected recovery apparatus. At least one first output hole 37a is arranged between the first recovery channel 50a and the first balloon chamber 311a, for example, the first output hole 37a is arranged on a side wall of the inner tube 33a. The first output hole 37a communicates the first recovery channel 50a and the first balloon chamber 311a and is configured to flow the cooling medium between the first recovery channel 50a and the first balloon chamber 311a. At least one second output hole 37b is arranged between the second recovery channel 50b and the second balloon chamber 311b, for example, the second output hole 37b is arranged on the side wall of the inner tube 33a. The second output hole 37b communicates the second recovery channel 50b and the second balloon chamber 311b and is configured to flow the cooling medium between the second recovery channel 50b and the second balloon chamber 311b. Apertures of the first output hole 37a and the second output hole 37b may not be too small, for example, the aperture of the first output hole 37a needs to be greater than or equal to the distal aperture of the first delivery channel 40a, and the aperture of the second output hole 37b needs to be greater than or equal to the distal aperture of the second delivery channel 40b to ensure smooth flow of the cooling medium therein. After the first balloon chamber 311a and the second balloon chamber 311b are inflated with the cooling medium and expanded, the first recovery channel 50a and the second recovery channel 50b are configured to output the cooling medium in the first balloon chamber 311a and the second balloon chamber 311b to the recovery apparatus, respectively.

During the process of ablation, the cooling balloon 30 in the shrinkage state is first inserted into the ablation portion 22, and the cooling apparatus delivers the cooling medium to the first balloon chamber 311a and the second balloon chamber 311b through the first delivery channel 40a and the second delivery channel 40b, respectively, so that the first balloon chamber 311a and the second balloon chamber 311b are in the expanded state, and the first outer wall 315a of the first balloon chamber 311a and the second outer wall 315b of the second balloon chamber 311b are attached to the target tissue. The cooling medium in the first balloon chamber 311a flows into the first recovery channel 50a through the first output hole 37a, and the cooling medium in the second balloon chamber 311b flows into the second recovery channel 50b through the second output hole 37b. The cooling medium in the first balloon chamber 311a and the second balloon chamber 311b is recovered to the connected recovery apparatus through the first recovery channel 50a and the second recovery channel 50b by applying a negative pressure to the first recovery channel 50a and the second recovery channel 50b, thereby constituting a cooling circuit. The construction of the cooling circuit facilitates continuous cooling of the target tissue and avoids excessive cooling medium entering the human body. The first balloon chamber 311a and the second balloon chamber 311b of the embodiment are both configured with independent cooling circuits so that the cooling property of the cooling circuits may be adjusted.

Further, the distance from the above-mentioned first output hole 37a to the distal end of the balloon main body 31 is less than the distance from the second output hole 37b to the proximal end of the balloon main body 31, and the distance from the above-mentioned second output hole 37b to the distal end of the balloon main body 31 is less than the distance from the second output hole 37b to the proximal end of the balloon main body 31. This arrangement allows the cooling medium to be recovered after sufficient contact with the tissue in the ablation area to maximally cool the target tissue.

The relatively flat dorsal muscular wall portion 12 in the airway is more susceptible to scarring during ablation than the "C" shaped region in the airway, and in order to better protect the epithelial tissue of the dorsal muscular wall portion 12, the cooling efficiency of the cooling balloon 30 at the dorsal muscular wall portion 12 needs to be improved. However, the cooling efficiency of the cooling balloon 30 is closely related to the flow rate of the cooling medium. The faster the flow rate of the cooling medium, the faster the speed of taking away the heat, and the slower the flow rate of the cooling medium, the slower the speed of taking away the heat. In the embodiment, when both the first balloon chamber 311a and the second balloon chamber 311b are in the expanded state, the delivery speed of the cooling medium in the first delivery channel 40a (i.e., the input speed of the cooling medium in the first balloon chamber 311a) is the same as the recovery speed of the cooling medium in the first recovery channel 50a (i.e., the output speed of the cooling medium in the first balloon chamber 311a). The delivery speed of the cooling medium in the second delivery channel 40b (i.e., the input speed of the cooling medium in the second balloon chamber 311b) is the same as the recovery speed of the cooling medium in the second recovery channel 50b (i.e., the output speed of the cooling medium in the second balloon chamber 311b). Further, the flow rate of the cooling medium in the second delivery channel 40b is greater than the flow rate of the cooling medium in the first delivery channel 40a, and the flow rate of the cooling medium in the second recovery channel 50b is greater than the flow rate of the cooling medium in the first recovery channel 50a.

When the cross-sectional area of the second balloon chamber 311b in an axial region where the second outer wall 315b is located is equal to the cross-sectional area of the first balloon chamber 311a in an axial region where the first outer wall 315a is located, the flow rate of the cooling medium in the axial region where the second outer wall 315b is located in the second balloon chamber 311b is greater than the flow rate of the cooling medium in the axial region where the first outer wall 315a is located in the first balloon chamber 311a, thereby making the cooling efficiency of the cooling balloon 30 at the second outer wall 315b higher than the cooling efficiency of the cooling balloon 30 at the first outer wall 315a.

Further, the balloon main body 31 of the embodiment may also refer to embodiment 8 so that the first cross-sectional area of the first balloon chamber 311a in the axial region where the first outer wall 315a is located is greater than the second cross-sectional area of the second balloon chamber 311b in the axial region where the second outer wall 315b is located.

For example, referring to Fig. 24, the axial regions where the first outer wall 315a and the second outer wall 315b of the cooling balloon 30 are located overlap, and the separating piece 38 includes a radially expandable and compressible inner balloon 31c and two laminar structures 38a. The inner balloon 31c is fixedly connected to the inner tube 33a, and a cooling cavity is formed between the outer surface of the inner balloon 31c and the inner surface of the balloon main body 31. The laminar structure 38a is located in the cooling cavity and separates the first balloon chamber 311a and the second balloon chamber 311b from the cooling cavity. For example, each side of the inner balloon 31c is provided with one laminar structure 38a, and one side edge of each laminar structure 38a is sealingly connected to the outer surface of the inner balloon 31c and the other side edge is sealingly connected to the balloon wall 315 of the balloon main body 31. The separating piece 38 separates the balloon chamber 311 into the first balloon chamber 311a and the second balloon chamber 311b. A third balloon chamber 311c of the above-mentioned inner balloon 31c and the second balloon chamber 311b may be jointly communicated with the same second delivery channel 40b, or a separate third delivery channel 40 may be arranged for the inner balloon 31c in the outer tube 32. Fig. 24 shows a schematic diagram of a cross-section of the balloon main body 31 within the axial regions where the first outer wall 315a and the second outer wall 315b are located. The cross-section of the balloon main body 31 has a circular profile, the cross-section of the inner balloon 31c has a fan-shaped profile, and the radius of the fan-shaped profile is less than the radius of the circular profile of the balloon main body 31. The cross-section of the laminar structure 38a has a linear profile, and the fan-shaped profile has an arcuate edge 315c. An endpoint of the arcuate edge 315c is connected to the linear profile of the laminar structure 38a. The fan-shaped profile and the two linear profiles separate the cross-section of the balloon main body 31 into three cross-sections, i.e., a first cross-section, a second cross-section, and a third cross-section, respectively. The first cross-section is a cross-section of the first balloon chamber 311a, the second cross-section is a cross-section of the second balloon chamber 311b, the third cross-section is a cross-section of the inner balloon 31c, and the area of the first cross-section is greater than the area of the second cross-section. This arrangement of the inner balloon 31c facilitates further compression of the cross-section of the second balloon chamber 311b to further increase the flow rate of the cooling medium in the second balloon chamber 311b, while saving the cooling medium to some extent.

### Embodiment 9

The embodiment proposes another cooling balloon 30 based on embodiments 5-8.

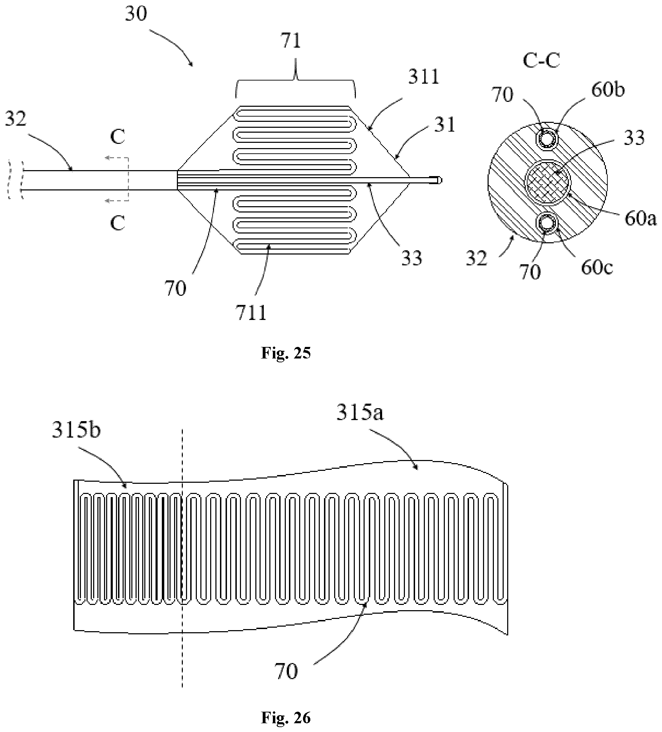

Referring to Figs. 25 and 26, the cooling balloon 30 of the embodiment includes an expandable balloon main body 31, a cooling pipe 70, and an outer tube 32 and an inner rod 33 fixedly connected to the balloon main body 31. The balloon main body 31 includes a balloon wall 315 and a balloon chamber 311 enclosed by the balloon wall 315. The balloon chamber 311 may be radially compressed and expanded. The balloon wall 315 includes a first outer wall 315a and a second outer wall 315b. The first outer wall 315a and the second outer wall 315b are arranged in the circumferential direction of the balloon main body 31 and are configured to attach to the target tissue when the balloon main body 31 is expanded. The distal end of the outer tube 32 is connected to a proximal end of the balloon main body 31, and the proximal end of the outer tube 32 connects the recovery apparatus through the operation handle 23. The inner rod 33 extends through the lumen of the outer tube 32. The proximal end of the inner rod 33 is connected to the operation handle 23, and the distal end of the inner rod 33 is located in the balloon main body 31 and is connected to the distal end of the balloon main body 31. The outer tube 32 is a multi-chamber tube, and its interior has a first accommodating passage 60a, a second accommodating passage 60b, and a third accommodating passage 60c independent of each other. The first accommodating passage 60a is configured to accommodate the inner rod 33, and the inner rod 33 extends through the accommodating passage. The outer diameter of the inner rod 33 is less than the diameter of the first accommodating passage 60a, and a delivery channel 40 may be formed between the inner rod 33 and the first accommodating passage 60a. The proximal end of the delivery channel 40 communicates with a connected delivery apparatus, and the distal end communicates with the balloon chamber 311 for delivering an inflation medium in the delivery device to the balloon chamber 311 to inflate and expand the balloon chamber 311. The delivery apparatus may be a cooling apparatus, and the inflation medium may be a cooling medium. In other embodiments, the delivery apparatus may deliver any other suitable inflation medium. The cooling pipe 70 has an input end portion, a recovery end portion (not shown in the figure), and a main body portion between the input end portion and the recovery end portion. The input end portion passes through the second accommodating passage 60b and connects the cooling apparatus through the operation handle 23 for delivering the cooling medium in the cooling apparatus into the cooling pipe 70. The recovery end portion passes through the third accommodating passage 60c and connects the recovery apparatus through the operation handle 23 for outputting the cooling medium in the cooling pipe 70 to the recovery apparatus.

In order to provide the above-mentioned cooling pipe 70 with radial expansion and shrinkage properties, the cooling pipe 70 may be made of the same material or a different material from that of the balloon main body 31, for example, one or a mixture of nylon, Pebax, polyurethane, polyethylene terephthalate, and polyethylene may be selected. In this embodiment, the main body portion of the cooling pipe 70 includes an encircling section 71. The encircling section 71 is provided around an outer surface of the balloon main body 31. For example, the encircling section 71 is fixedly connected to the outer surface of the balloon main body 31 (e.g., in an integral structure with the balloon main body 31) and is configured to attach to the target tissue to cool the target tissue. In other embodiments, the encircling section 71 may be provided in the balloon main body 31 and tightly attached to an inner surface of the balloon main body 31.

The encircling section 71 includes a plurality of main body sections 711 arranged along a circumferential direction of the balloon main body 31 (e.g., a plurality of main body sections 711 arranged in parallel), and the plurality of main body sections 711 are connected end-to-end in the circumferential direction. The main body section 711 is linear and has a head end and a tail end, with the head end being closer to the outer tube 32 than the tail end. In other embodiments, the main body section 711 may also be wave-shaped, arcuate, and other shapes. In addition, in this embodiment, the cross-sectional shape of the main body section 711 is circular, and in other embodiments, the cross-sectional shape of the main body section 711 may also be oval, quadrangular, etc. When the cross-sectional shape of the main body section 711 is quadrangular, it is advantageous to increase an attachment area between the main body section 711 and the target tissue to improve the cooling efficiency.

During the process of ablation, the cooling balloon 30 in the shrinkage state is first inserted into the ablation portion 22, and the delivery apparatus delivers the inflation medium to the balloon main body 31 through the delivery channel 40 so that the balloon main body 31 is in the expanded state. At the same time, the cooling apparatus continuously inputs the cooling medium to the input end portion of the cooling pipe 70 so that the cooling pipe 70 is inflated and expanded, and the encircling section 71 is attached to the target tissue. The cooling medium in the cooling pipe 70 is recovered to the connected recovery apparatus through the output end portion by applying a negative pressure to the recovery channel 50, thereby constituting a cooling circuit. The construction of the cooling circuit facilitates continuous cooling of the target tissue and avoids excessive cooling medium entering the human body.

The relatively flat dorsal muscular wall portion 12 in the airway is more susceptible to scarring during ablation than the "C" shaped region in the airway, and in order to better protect the epithelial tissue of the dorsal muscular wall portion 12, the cooling efficiency of the cooling balloon 30 at the dorsal muscular wall portion 12 needs to be improved. In this embodiment, the second outer wall 315b is configured to attach to the dorsal muscular wall portion 12, while the first outer wall 315a is configured to attach to the "C" shaped region of the airway. The concentration of the cooling pipe 70 on the first outer wall 315a is less than the concentration of the cooling pipe 70 on the second outer wall 315b so that the cooling efficiency of the cooling balloon 30 at the second outer wall 315b is higher than the cooling efficiency of the cooling balloon 30 at the first outer wall 315a. A method for testing the above-mentioned concentration includes the following steps. A balloon wall 315 to be tested (the first outer wall 315a or the second outer wall 315b) and the cooling pipe 70 covered thereon are cut down, and the balloon wall 315 to be tested is deployed flat in a plane. An area S1 enclosed by an outer profile of the balloon wall 315 to be tested in a deployed state is measured, and further, a sum S2 of orthographic projection areas of the outermost cooling pipe 70 on the balloon wall 315 to be tested in the plane is measured. The ratio (i.e., (S2/S1)) of the sum of the orthographic projection areas of the outermost cooling pipe 70 on the balloon wall 315 to be tested in the plane and the area enclosed by the outer profile of the balloon wall 315 to be tested is the concentration.

For the arrangement of the cooling pipe 70 as shown in Fig. 26, the main body sections 711 on the balloon main body 31 are all arranged in parallel and at uniform intervals, and pipe diameters of the main body sections 711 are substantially equal. The concentration of the cooling pipe 70 on the first outer wall 315a and the second outer wall 315b may be compared by measuring gaps between adjacent main body sections 711 on the first outer wall 315a and the second outer wall 315b. For example, the greater the gap width between adjacent main body sections 711, the smaller the concentration of the cooling pipe 70, and the smaller the gap width between adjacent main body sections 711, the greater the concentration of the cooling pipe 70. As can be seen from Fig. 26, in this embodiment, the gap width between adjacent main body sections 711 on the first outer wall 315a is greater than the gap width between adjacent main body sections 711 on the second outer wall 315b.

It can be understood that the cooling balloons 30 in the above-mentioned embodiments 5-9 are not limited to be used in combination with the ablation portions 22 in embodiments 1-4, and in other embodiments, the cooling balloon 30 may also be used in combination with ablation portions 22 with other shapes and structures. For example, one or more electrodes 25, i.e., the ablation portions 22, are fixedly provided on the outer surface of the cooling balloon 30, and the electrode 25 connects an energy generator, which can also achieve the effect of ablating the target tissue.

The above are only preferred specific implementations of the present disclosure, but the scope of the present disclosure is not limited thereto. Any changes or substitutions that may be easily thought of by a person skilled in the art within the technical scope disclosed in the present disclosure shall be covered by the scope of the present disclosure. Therefore, the scope of the present disclosure shall be subject to the scope of the claims.

## Claims

1. An ablation apparatus, comprising: a catheter and an ablation portion connected to a distal end of the catheter, wherein the ablation portion has an expansible property and comprises a plurality of ablation members; each ablation member comprises a supporting unit, a wall attachment unit, and an energy release unit provided on the wall attachment unit; a plurality of supporting units are deployed radially outwardly and extend toward the distal end of the ablation portion to be connected to a plurality of wall attachment units; when the ablation portion is in a naturally expanded state, at least one wall attachment unit in the ablation portion is independently deformed relative to the adjacent wall attachment units.

2. The ablation apparatus according to claim 1, wherein the plurality of supporting units is enclosed to form a cavity having an opening at a distal end, and the plurality of wall attachment units are provided around the opening at spaced intervals.

3. The ablation apparatus according to claim 1, wherein the wall attachment unit comprises a first connecting end and a second connecting end connected to the supporting unit, and a deformable section extending between the first connecting end and the second connecting end; the deformable section has an elastic deformation property, and when the ablation portion is in the naturally expanded state, the deformable section is bent and protruded toward an outer side of the ablation portion relative to the first connecting end and the second connecting end.

4. The ablation apparatus according to claim 3, wherein the deformable section is elastically deformed to increase a distance between the first connecting end and the second connecting end when the deformable section is subjected to a force toward an inside of the ablation portion.

5. The ablation apparatus according to claim 3, wherein the deformable section comprises a first wall attachment section and a second wall attachment section; one end of the first wall attachment section is connected to the first connecting end, the other end of the first wall attachment section is connected to one end of the second wall attachment section, and the other end of the second wall attachment section is connected to the second connecting end; the first connecting end and the second connecting end are configured to be close to each other under the action of an external force and configured to be away from each other when the external force is removed.

6. The ablation apparatus according to claim 5, wherein the first wall attachment section and the second wall attachment section each comprises an arcuate structure protruding toward the outer side of the ablation portion, or the first wall attachment section and the second wall attachment section each comprises a straight-line structure inclined toward the outer side of the ablation portion; or, one of the first wall attachment section and the second wall attachment section comprises the arcuate structure protruding toward the outer side of the ablation portion, and the other comprises the straight-line structure inclined toward the outer side of the ablation portion.

7. The ablation apparatus according to claim 5, wherein the deformable section further comprises a connecting section; the connecting section comprises a distal vertex, and a first connecting arm and a second connecting arm jointly connecting the distal vertex; a gap exists between the proximal end of the first connecting arm and the proximal end of the second connecting arm; one end of the first wall attachment section is connected to the first connecting end, and the other end of the first wall attachment section is connected to the first connecting arm; one end of the second wall attachment section is connected to the second connecting arm, and the other end of the second wall attachment section is connected to the second connecting end.

8. The ablation apparatus according to claim 7, wherein the connecting section protrudes toward the distal end of the ablation apparatus.

9. The ablation apparatus according to claim 3, wherein the supporting unit comprises two first supporting rods provided at spaced intervals, and the first connecting end and the second connecting end are fixedly connected to the two first supporting rods, respectively.

10. The ablation apparatus according to claim 9, wherein the ablation portion further comprises a supporting member; the supporting member comprises a plurality of second supporting rods arranged at spaced intervals; the proximal end of each second supporting rod is fixedly connected to the catheter, and the distal end of each second supporting unit is fixedly connected to two adjacent first supporting units.

11. The ablation apparatus according to claim 1, wherein the supporting unit comprises a straight section and is connected to the wall attachment unit through the straight section; the included angle between the straight section and an axis of the ablation portion is less than the included angle between a remaining region of the supporting unit except the straight section and the axis of the ablation portion in a naturally expanded state.

12. The ablation apparatus according to claim 1, wherein the ablation apparatus has a first region and a second region; the wall attachment unit in the first region is independently deformed relative to the adjacent wall attachment units, and the wall attachment units in the first region and the wall attachment units in the second region are located on the same plane, or the wall attachment units in the first region and the wall attachment units in the second region are located in different planes.

13. The ablation apparatus according to claim 12, wherein the wall attachment units in the first region are located between the wall attachment units in the second region and the catheter, and when both the first region and the second region are subjected to a force toward an inside of the ablation portion, at least one wall attachment unit in the second region abuts against an inner side of the wall attachment unit in the first region.

14. The ablation apparatus according to claim 12, wherein the energy release unit located in the first region comprises an electrode pair and/or strip electrodes; the electrode pair comprises a first electrode and a second electrode of opposite polarities, and the first electrode and the second electrode are provided at spaced intervals on one or more wall attachment units; the strip electrodes extend along a longitudinal direction of the wall attachment unit.

15. The ablation apparatus according to claim 1, wherein the wall attachment unit comprises a sheet-like mesh structure; the mesh structure comprises conductive wires, and the conductive wire is exposed to form a mesh electrode.

16. The ablation apparatus according to claim 1, wherein the catheter and the ablation portion have delivery channels that communicate with each other; and/or the distal end of the catheter is provided with an extension tube, the extension tube extends through the ablation portion, and the catheter and the extension tube are provided with delivery channels that communicate with each other; first cooling holes are arranged on a surface of the ablation portion and/or the extension tube, and the first cooling hole communicates the delivery channel with the outside; the delivery channel is configured to deliver a cooling medium, and the first cooling hole is configured to release the cooling medium to the outside.

17. The ablation apparatus according to claim 1, further comprising a cooling balloon, wherein the cooling balloon comprises a balloon main body having a balloon chamber, a delivery channel communicating with the balloon chamber; the balloon main body is provided in a chamber of the ablation portion, and when the balloon main body is in an expanded state, an outer surface of the balloon main body is attached to the wall attachment unit; the delivery channel is configured to deliver a cooling medium into the balloon chamber; one or more second cooling holes are arranged on a surface of the cooling balloon and are configured to communicate the balloon chamber with the outside and discharge the cooling medium in the balloon chamber to the outside.

18. The ablation apparatus according to claim 17, wherein the cooling balloon further comprises a recovery channel communicated with the balloon chamber, and the recovery channel is configured to recover the cooling medium from the balloon chamber.

19. A cooling balloon, comprising: an expandable balloon main body, a delivery channel, and a recovery channel, wherein the balloon main body comprises a first balloon chamber and a second balloon chamber arranged in a radial direction, and the delivery channel, the recovery channel, the first balloon chamber, and the second balloon chamber form a cooling circuit for a cooling medium to flow; the first balloon chamber has a first outer wall, the second balloon chamber has a second outer wall, and the first outer wall and the second outer wall are arranged in a circumferential direction of the balloon main body; any cross-sectional area of the first balloon chamber in an axial region where the first outer wall is located is a first cross-sectional area, any cross-sectional area of the second balloon chamber in an axial region where the second outer wall is located is a second cross-sectional area, and the first cross-sectional area is greater than the second cross-sectional area.

20. The cooling balloon according to claim 19, further comprising an outer tube, a separating piece, and an output hole, wherein the distal end of the outer tube is connected to the proximal end of the balloon main body; the outer tube is a multi-chamber tube, and the interior of the outer tube has the delivery channel and the recovery channel independent of each other; one of the first balloon chamber and the second balloon chamber communicates with the distal end of the delivery channel, and the other communicates with the distal end of the recovery channel; the proximal end of the delivery channel is configured to connect a cooling apparatus, and the proximal end of the recovery channel is configured to connect a recovery apparatus; the separating piece is fixedly connected to an interior of the balloon main body and is provided between the first balloon chamber and the second balloon chamber, configured to separate the first balloon chamber and the second balloon chamber; at least one output hole is arranged between the first balloon chamber and the second balloon chamber, and the output hole communicates the first balloon chamber and the second balloon chamber and is configured to flow the cooling medium between the first balloon chamber and the second balloon chamber.

21. The cooling balloon according to claim 20, wherein the separating piece comprises a laminar structure; the laminar structure extends from the proximal end to the distal end of the balloon main body to separate the first balloon chamber and the second balloon chamber, and the cross-sectional area occupied by the first balloon chamber is greater than the cross-sectional area occupied by the second balloon chamber in any cross-section of the balloon main body in the axial region where the separating piece is located.

22. The cooling balloon according to claim 21, wherein the separating piece further comprises an inner balloon, and a cooling cavity is formed between an outer surface of the inner balloon and an inner surface of the balloon main body; the laminar structure is located in the cooling cavity and separates the first balloon chamber and the second balloon chamber from the cooling cavity.

23. The cooling balloon according to claim 20, further comprising an inner rod, wherein an accommodating passage is also provided in the outer tube; the inner rod extends through the accommodating passage, and the distal end of the inner rod extends into the balloon chamber and is fixedly connected to the distal end of the balloon main body.

24. The cooling balloon according to claim 21, wherein the output hole is located on the separating piece and/or on an inner rod, and the distance from the output hole to the distal end of the balloon main body is less than the distance from the output hole to the proximal end of the balloon main body.

25. The cooling balloon according to claim 19, wherein the delivery channel comprises a first delivery channel and a second delivery channel, and the recovery channel comprises a first recovery channel and a second recovery channel; the first delivery channel, the first recovery channel, and the first balloon chamber form a first cooling circuit, and the second delivery channel, the second recovery channel, and the second balloon chamber form a second cooling circuit; when the first balloon chamber and the second balloon chamber are in an expanded state, the flow rate of the cooling medium in the axial region where the second outer wall of the second balloon chamber is located is greater than the flow rate of the cooling medium in the axial region where the first outer wall of the first balloon chamber is located.

26. The cooling balloon according to claim 19, wherein the surface roughness of the inner surface of the second outer wall is less than the surface roughness of the inner surface of the first outer wall.

27. The cooling balloon according to claim 19, wherein the outer surface hardness of the second outer wall is less than the outer surface hardness of the first outer wall.

28. The cooling balloon according to claim 19, wherein the balloon main body is provided with a cooling pipe, the concentration of the cooling pipe on the first outer wall is less than the concentration of the cooling pipe on the second outer wall.

29. An ablation apparatus, comprising the cooling balloon according to any one of claims 19 to 28 and an ablation portion, wherein the ablation portion is configured to ablate a target tissue; the cooling balloon is inserted into the ablation portion and is configured to cool the target tissue.
